(19) Europäisches Patentamt
European Patent Office
Office européen des brevets

(11) EP 1 956 036 A1

(12) **EUROPEAN PATENT APPLICATION**
published in accordance with Art. 158(3) EPC

(43) Date of publication:
**13.08.2008 Bulletin 2008/33**

(21) Application number: **06833143.8**

(22) Date of filing: **22.11.2006**

(51) Int Cl.:
***C08G 64/30*** *(2006.01)* ***B01D 3/14*** *(2006.01)*
***B01D 3/22*** *(2006.01)* ***C07C 68/06*** *(2006.01)*
***C07C 69/96*** *(2006.01)*

(86) International application number:
**PCT/JP2006/323321**

(87) International publication number:
**WO 2007/063757 (07.06.2007 Gazette 2007/23)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.11.2005 JP 2005346770**

(71) Applicant: **Asahi Kasei Chemicals Corporation**
**Tokyo 100-8440 (JP)**

(72) Inventors:
- **FUKUOKA, Shinsuke**
  **Tokyo, 100-8440 (JP)**
- **MIYAJI, Hironori**
  **Tokyo, 100-8440 (JP)**
- **HACHIYA, Hiroshi**
  **Tokyo, 100-8440 (JP)**
- **MATSUZAKI, Kazuhiko**
  **Tokyo, 100-8440 (JP)**

(74) Representative: **Strehl Schübel-Hopf & Partner**
**Maximilianstrasse 54**
**80538 München (DE)**

(54) **INDUSTRIAL PRODUCTION PROCESS FOR HIGH-QUALITY AROMATIC POLYCARBONATE**

(57) It is an object of the present invention to provide a specific process that enables a high-quality high-performance aromatic polycarbonate having excellent mechanical properties and no discoloration to be produced industrially in a large amount (e.g. not less than 1 ton / hr) stably for a prolonged period of time (e.g. not less than 1000 hours, preferably not less than 3000 hours, more preferably not less than 5000 hours) from a dialkyl carbonate and an aromatic dihydroxy compound. When producing the aromatic polycarbonate from the dialkyl carbonate and the aromatic dihydroxy compound, the above object can be attained by carrying out a process of the present invention which comprises the step of; (I) producing a diaryl carbonate using two reactive distillation columns each having a specified structure: (II) purifying the diaryl carbonate so as to obtain a high-purity diaryl carbonate: (III) subsequently producing an aromatic polycarbonate using a guide-contacting downflow type polymerization apparatus having a specified structure from a molten prepolymer obtained from an aromatic dihydroxy compound and the high-purity diaryl carbonate: and (IV) recycling by-produced aromatic monohydroxy compound into the step (I).

**FIG.5**

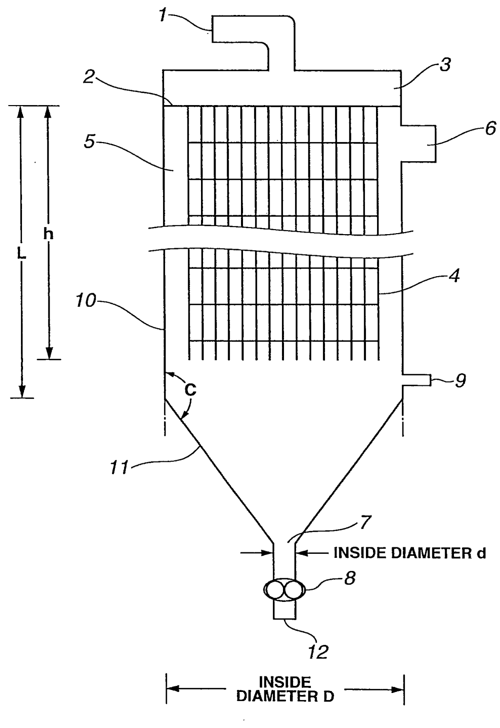


EP 1 956 036 A1

Printed by Jouve, 75001 PARIS (FR)

## Description

### Technical Field

The present invention relates to an industrial process for the production of an aromatic polycarbonate. More particularly, the present invention relates to a process for industrially mass-producing a high-quality high-performance aromatic polycarbonate having excellent mechanical properties and no coloration stably for a prolonged period of time from a dialkyl carbonate and an aromatic dihydroxy compound.

### Background Art

Aromatic polycarbonates are widely used in many fields as engineering plastics having excellent heat resistance, impact resistance, transparency and so on. A variety of studies have been carried out hitherto into processes for producing such aromatic polycarbonates, and of these, an interfacial polycondensation process between phosgene and an aromatic dihydroxy compound such as 2,2-bis(4-hydroxyphenyl)propane (hereinafter referred to as "bisphenol A") has been industrialized. However, there are many problems with this interfacial polycondensation process, for example toxic phosgene must be used, methylene chloride, which has health and environmental problems, must be used as a polymerization solvent in a large amount of at least 10 times that of the polycarbonate, the apparatus is corroded by chlorine-containing compounds such as methylene chloride and by-produced hydrogen chloride and sodium chloride, separating out residual chlorinated impurities such as sodium chloride and methylene chloride that have an adverse effect on the polymer properties is difficult, and treatment of a large amount of process wastewater containing methylene chloride, unreacted bisphenol A and so on is necessary.

On the other hand, as a process for producing aromatic polycarbonates from aromatic dihydroxy compounds and diaryl carbonates, a melt process in which, for example, bisphenol A and diphenyl carbonate are subjected to transesterification in a molten state, and polymerization is carried out while removing by-produced phenol has been known from hitherto. This transesterification reaction is an equilibrium reaction, and moreover the equilibrium constant is low, and hence the polymerization will not proceed unless the phenol is removed efficiently from the surface of the molten matter. Unlike the interfacial polycondensation process, the melt process has advantages such as a solvent not being used, but on the other hand there has been a problem intrinsic to aromatic polycarbonates that once the polymerization has proceeded to a certain extent the viscosity of the polymer increases rapidly, and hence it becomes difficult to remove by-produced phenol and so on out of the reaction system efficiently, whereby it becomes impossible to substantially increase the polymerization degree. That is, in the case of the aromatic polycarbonates, unlike in the case of molten polycondensation to produce another condensation polymer such as polyamides or polyesters, the melt viscosity becomes extremely high even in a low molecular weight state, for example at a polymerization degree (n) of 15 to 20, and hence surface renewal becomes very difficult using ordinary stirring. Removal of phenol from the polymer surface thus substantially stops occurring, and hence a polymer having a polymerization degree required for a product (n approximately 33 to 65) cannot be produced. This is well known in the person skilled in the art.

As polymerization apparatuses for producing the aromatic polycarbonates using such a melt process, various polymerization apparatuses are known. A process using a vertical stirring tank type polymerization apparatus having a stirrer is widely known. However, with such a vertical stirring tank type polymerization apparatus, in the case of small scale production, the volumetric efficiency is high, and there is an advantage of the apparatus being simple, and hence the polymerization can be made to proceed efficiently. However, in the case of industrial scale production, it is difficult to efficiently remove out of the system phenol that is by-produced as the polymerization proceeds as described above, and hence there is the problem that the polymerization rate is very low. Furthermore, for a large scale vertical stirring tank type polymerization apparatus, a ratio of the liquid volume to the evaporation area is generally greater than in the case of a small scale, the state being such that the so-called liquid depth is high. Consequently, even if the degree of vacuum is increased so as to increase the polymerization degree, because there is a high liquid depth in the lower portion of the stirring tank, the polymerization takes place at a higher pressure corresponding to the liquid depth than in the space in the upper portion of the stirring tank, and hence efficiently removing phenol or the like is difficult. The large scale vertical stirring tank type polymerization apparatus can thus only be used in the case of producing a prepolymer. To attain the required polymerization degree, a polymerization apparatus for further subjecting this prepolymer to condensation polymerization is essential.

Various means have been used for efficiently removing phenol or the like from the high-viscosity polymer in order to solve the above problem. Most of these means have related to improving the mechanical stirring, for example there have been described a process using a screw type polymerization apparatus having a vent (see Patent Document 1: Japanese Patent Publication No. S50-19600 (corresponding to Brit. Patent No. 1007302)), a process using an interlocking type twin screw extruder (see Patent Document 2: Japanese Patent Publication No. S52-36159), and a process using a thin film evaporation type reactor such as a screw evaporator or a centrifugal thin film evaporator (see Patent

Document 3: Japanese Patent Publication No. S53-5718 (corresponding to U.S. Patent No. 3,888,826)). In addition, there has been specifically disclosed a process using a combination of a centrifugal thin film evaporator and a horizontal biaxial stirring type polymerization apparatus (see Patent Document 4: Japanese Patent Application Laid-open No. H2-153923). These processes all have carrying out mechanical stirring as the basis of the art, and hence there are naturally limitations, and thus the above problems have not been solved.

That is, there are limitations on mechanical stirring itself in terms of being able to handle ultra-high melt viscosity, and hence various problems relating to the ultra-high melt viscosity of aromatic polycarbonates have remained unresolved. With the above processes, it has been attempted to resolve these problems by raising the temperature so as to lower the melt viscosity at least a little. That is, in these processes, the molten prepolymer is subjected to polymerization at a high temperature of around 300 °C and under a high vacuum while trying to bring about surface renewal through mechanical stirring, but even at this temperature the melt viscosity is still very high, and hence the extent of surface renewal cannot be made high. There are thus limitations on the polymerization degree of polycarbonates that can be produced using these processes, it being difficult to produce a high molecular weight grade product. Furthermore, with these processes, discoloration or deterioration in properties of the polymer obtained is liable to occur due to the reaction being carried out at a high temperature of around 300 °C, and moreover such discoloration or deterioration in properties of the polymer is also liable to occur due to air or foreign matter leaking in through a vacuum seal part of the stirring apparatus. There are thus still many problems to be solved in order to produce a high-quality polycarbonate stably for a prolonged period of time.

The present inventors have discovered that these problems can be completely solved by developing a process in which mechanical stirring is not carried out, but rather a guide-contacting downflow type polymerization apparatus is used in which the molten prepolymer is subjected to polymerization while being made to fall down under its own weight along guides such as wires, and have previously filed patent applications (see, for example, Patent Document 5: Japanese Patent Application Laid-open No. H 8-225641, Patent Document 6: Japanese Patent Application Laid-open No. H8-225643, Patent Document 7: Japanese Patent Application Laid-open No. H8-325373, Patent Document 8: WO 97-22650, Patent Document 9: Japanese Patent Application Laid-open No. H10-81741, Patent Document 10: Japanese Patent Application Laid-open No. H10-298279, Patent Document 11: WO 99/36457, Patent Document 12: WO 99/64492). However, with such processes, there has been no disclosure or suggestion of a specific industrial production process that enables not less than 1 ton / hr of an aromatic polycarbonate to be produced.

Furthermore, to produce the aromatic polycarbonates on an industrial scale through transesterification, the high-purity diaryl carbonates must be procured in a large amount on an industrial scale. Aromatic dihydroxy compounds such as high-purity bisphenol A are mass-produced industrially, and can easily be procured, but the high-purity diaryl carbonates cannot be procured in a large amount on an industrial scale. It is thus necessary to produce the high-purity diaryl carbonates.

As a process for producing the diaryl carbonates, a process of reacting the aromatic monohydroxy compounds with phosgene has been known from long ago, and has also been the subject of a variety of studies in recent years. However, this process has the problem of using phosgene, and in addition chlorinated impurities that are difficult to separate out are present in the diaryl carbonates produced using this process, and hence the diaryl carbonates cannot be used as a starting material for the production of the aromatic polycarbonates as is. The reason for this is that such chlorinated impurities markedly inhibit the polymerization reaction in the transesterification method for producing the aromatic polycarbonates which are carried out in the presence of an extremely small amount of a basic catalyst; for example, even if such chlorinated impurities are present in an amount of only 1 ppm, the polymerization hardly proceeds at all. To make the diaryl carbonates capable of being used as the starting material of a transesterification method aromatic polycarbonate, a troublesome multi-stage separation / purification process involving thorough washing with a dilute aqueous alkaline solution and hot water, oil / water separation, distillation and so on is thus required. Furthermore, the yield decreases due to hydrolysis loss and distillation loss during this separation / purification process. There are thus many problems in carrying out this process economically on an industrial scale.

On the other hand, a process for producing aromatic carbonates through transesterification reactions between the dialkyl carbonates and the aromatic monohydroxy compounds is also known. However, such transesterification reactions are all equilibrium reactions. The equilibrium is biased extremely toward the original system and the reaction rate is slow, and hence there have been many difficulties in producing aromatic carbonates industrially in large amounts using such a process.

Several proposals have been made to improve on the above difficulties, but most of these have related to development of a catalyst to increase the reaction rate. Many metal compounds have been proposed as catalysts for this type of transesterification reaction. However, the problem of the disadvantageous equilibrium cannot be resolved merely by developing a catalyst, and hence there are very many issues to be resolved including the reaction system in order to provide a process for industrial production aiming for mass production.

Attempts have also been made to devise a reaction system so as to shift the equilibrium toward the product system as much as possible, and thus improve the aromatic carbonate yield. For example, for the reaction between

dimethyl carbonate and phenol, there have been proposed a method in which methanol produced as a by-product is distilled off by azeotropy together with an azeotrope-forming agent (see Patent Document 13: Japanese Patent Application Laid-Open No. S54-48732 (corresponding to West German Patent Application Laid-Open No. 736063, and U.S. Patent No. 4252737)), and a method in which the methanol produced as a by-product is removed by being adsorbed onto a molecular sieve (see Patent Document 14: Japanese Patent Application Laid-Open No. S58-185536 (corresponding to U.S. Patent No. 410464)). Moreover, a method has also been proposed in which, using an apparatus in which a distillation column is provided on top of a reactor, an alcohol produced as a by-product in the reaction is separated off from the reaction mixture, and at the same time unreacted starting material that evaporates is separated off by distillation (see Patent Document 15: Japanese Patent Application Laid-Open No. S56-123948 (corresponding to U.S. Patent No. 4182726)).

However, these reaction systems are basically batch system or switchover system. This is because there is a limitation on how much the reaction rate can be improved through catalyst development for such a transesterification reaction, and hence the reaction rate is still slow, and thus it has been thought that a batch system is preferable to a continuous system. Of these, a continuous stirring tank reactor (CSTR) system in which a distillation column is provided on top of a reactor has been proposed as a continuous system, but there are problems such as the reaction rate being slow, and the gas-liquid interface in the reactor being small based on the volume of the liquid. It is thus not possible to make the conversion high. Accordingly, it is difficult to attain the object of producing the aromatic carbonates continuously in large amounts stably for a prolonged period of time by means of the above methods, and many issues remain to be resolved before economical industrial implementation is possible.

The present inventors have developed reactive distillation methods in which such a transesterification reaction is carried out in a continuous multi-stage distillation column simultaneously with separation by distillation, and have been the first in the world to disclose that such a reactive distillation system is useful for such a transesterification reaction, for example a reactive distillation method in which the dialkyl carbonates and the aromatic hydroxy compounds are continuously fed into the multi-stage distillation column, and reaction is carried out continuously inside the column in which a catalyst is present, while continuously withdrawing by distillation a low boiling point component containing alcohols produced as a by-product, and continuously withdrawing a component containing produced alkyl aryl carbonates from a lower portion of the column (see Patent Document 16: Japanese Patent Application Laid-Open No. H3-291257), a reactive distillation method in which alkyl aryl carbonates are continuously fed into a multi-stage distillation column, and reaction is carried out continuously inside the column in which a catalyst is present, while continuously withdrawing by distillation a low boiling point component containing dialkyl carbonates produced as a by-product, and continuously withdrawing a component containing produced diaryl carbonates from a lower portion of the column (see Patent Document 17: Japanese Patent Application Laid-Open No. H4-9358), a reactive distillation method in which these reactions are carried out using two continuous multi-stage distillation columns, and hence the diaryl carbonates are produced continuously while efficiently recycling the dialkyl carbonates produced as a by-product (see Patent Document 18: Japanese Patent Application Laid-Open No. H4-211038 (corresponding to WO 91/09832, European Patent No. 0461274, and U.S. Patent No. 5210268)), and a reactive distillation method in which the dialkyl carbonates and the aromatic hydroxy compounds or the like are continuously fed into the multi-stage distillation column, and a liquid that flows down through the column is withdrawn from a side outlet provided at an middle stage and / or a lowermost stage of the distillation column, and is introduced into a reactor provided outside the distillation column so as to bring about reaction, and is then introduced back in through a circulating inlet provided at a stage above the stage where the outlet is provided, whereby reaction is carried out in both the reactor and the distillation column (see Patent Document 19: Japanese Patent Application Laid-Open No. H4-235951).

These reactive distillation methods proposed by the present inventors are the first to enable aromatic carbonates to be produced continuously and efficiently, and processes in which the diaryl carbonates are produced from the dialkyl carbonates using two continuous multi-stage distillation columns based on the above disclosures have been proposed thereafter (see, for example, Patent Document 20: Japanese Patent Application Laid-Open No. H6-157424 (corresponding to European Patent No. 0582931, and U.S. Patent No. 5334742), Patent Document 21: Japanese Patent Application Laid-Open No. H6-184058 (corresponding to European Patent No. 0582930, and U.S. Patent No. 5344954), Patent Document 22: Japanese Patent Application Laid-Open No. H9-40616, Patent Document 23: Japanese Patent Application Laid-Open No. H9-59225, Patent Document 24: Japanese Patent Application Laid-Open No. H9-176094, Patent Document 25: WO 00/18720 (corresponding to U.S. Patent No. 6093842), Patent Document 26: Japanese Patent Application Laid-Open No. 2001-64235).

Among reactive distillation systems, the present applicant has further proposed, as a method that enables highly pure aromatic carbonates to be produced stably for a prolonged period of time without a large amount of a catalyst being required, a method in which high boiling point material containing a catalyst component is reacted with an active substance and then separated off, and the catalyst component is recycled (see Patent Document 27: WO 97/11049 (corresponding to European Patent No. 0855384, and U.S. Patent No. 5872275)), and a method carried out while keeping a weight ratio of a polyhydric aromatic hydroxy compound in the reaction system to a catalyst metal at not more than

2.0 (see Patent Document 28: Japanese Patent Application Laid-Open No. H11-92429 (corresponding to European Patent No. 1016648, and U.S. Patent No. 6262210)). Furthermore, the present inventors have proposed a method in which 70 to 99 % by weight of phenol produced as a by-product in a polymerization process is used as a starting material, and a diphenyl carbonate are produced using a reactive distillation method; this diphenyl carbonate can be used as a starting material for polymerization to produce the aromatic polycarbonates (see Patent Document 29: Japanese Patent Application Laid-open No. H9-255772 (corresponding to European Patent No. 0892001, and U.S. Patent No. 5747609)).

However, in all of these prior art documents in which the production of aromatic carbonates using the reactive distillation method is proposed, there is no disclosure whatsoever of a specific process or apparatus enabling mass production on an industrial scale (e.g. 1 ton / hr), nor is there any description suggesting such a process or apparatus. For example, the descriptions regarding heights ($H_1$ and $H_2$: cm), diameters ($D_1$ and $D_2$: cm), numbers of stages ($n_1$ and $n_2$), and starting material feeding rates ($Q_1$ and $Q_2$: kg / hr) for two reactive distillation columns disclosed for producing mainly diphenyl carbonate (DPC) from dimethyl carbonate and phenol are as summarized in Table 1.

[Table 1]

**TABLE 1**

| $H_1$ | $D_1$ | $n_1$ | $Q_1$ | $H_2$ | $D_2$ | $n_2$ | $Q_2$ | PATENT DOCUMENTS |
|---|---|---|---|---|---|---|---|---|
| 600 | 25 | 20 | 66 | 600 | 25 | 20 | 23 | 18 |
| 350 | 2.8 | - | 0.2 | 305 | 5~10 | 15+ PACKINGS | 0.6 | 21 |
| 500 | 5 | 50 | 0.6 | 400 | 8 | 50 | 0.6 | 22 |
| 100 | 4 | - | 1.4 | 200 | 4 | - | 0.8 | 23 |
| 300 | 5 | 40 | 1.5 | | 5 | 25 | 0.7 | 24 |
| 1200 | 20 | 40 | 86 | 600 | 25 | 20 | 31 | 27<br>28 |
| 600 | | 20 | 66 | 600 | | 20 | 22 | 29 |

In other words, the biggest pairs of continuous multi-stage distillation columns used when carrying out this reaction using the reactive distillation system are those disclosed by the present applicants in Patent Document 27 (WO 97/11049 (corresponding to European Patent No. 0855384, and U.S. Patent No. 5872275)) and Patent Document 28 (Japanese Patent Application Laid-Open No. H11-92429 (corresponding to European Patent No. 1016648, and U.S. Patent No. 6262210)). As can be seen from Table 1, the maximum values of the various conditions for the continuous multi-stage distillation columns disclosed for the above reaction are $H_1$ = 1200 cm, $H_2$ = 600 cm, $D_1$ = 20 cm, $D_2$ = 25 cm, $n_1 = n_2$ = 50 (only this condition, Patent Document 22 (Japanese Patent Application Laid-Open No. H9-40616)), $Q_1$ = 86 kg / hr, and $Q_2$ = 31 kg / hr, and the amount of diphenyl carbonate produced has not exceeded approximately 6.7 kg / hr, which is not an amount produced on an industrial scale.

Disclosure of Invention

Problems to be solved by the invention

It is an object of the present invention to provide a specific process that enables a high-quality high-performance aromatic polycarbonate having excellent mechanical properties and no discoloration to be produced industrially in a large amount (e.g. not less than 1 ton / hr) stably for a prolonged period of time (e.g. not less than 1000 hours, preferably not less than 3000 hours, more preferably not less than 5000 hours) from a dialkyl carbonate and an aromatic dihydroxy compound.

Means for solving the problems

The present inventors have carried out studies aimed at discovering a specific process enabling the above object to be attained, and as a result have reached to the present invention. That is, the present invention provides:

1. an industrial process for the production of a high-quality aromatic polycarbonate in which an aromatic polycarbonate is continuously produced from a dialkyl carbonate and an aromatic dihydroxy compound, the process comprising the steps of:

(I) continuously producing a diaryl carbonate by taking the dialkyl carbonate and the aromatic monohydroxy compound as a starting material, continuously feeding the starting material into a first continuous multi-stage distillation column in which a catalyst is present, carrying out reaction and distillation simultaneously in said first column, continuously withdrawing a first column low boiling point reaction mixture containing a produced alcohol from an upper portion of said first column in a gaseous form, continuously withdrawing a first column high boiling point reaction mixture containing a produced alkyl aryl carbonate from a lower portion of said first column in a liquid form, continuously feeding said first column high boiling point reaction mixture into a second continuous multi-stage distillation column in which a catalyst is present, carrying out reaction and distillation simultaneously in said second column, continuously withdrawing a second column low boiling point reaction mixture containing a produced dialkyl carbonate from an upper portion of said second column in a gaseous form, continuously withdrawing a second column high boiling point reaction mixture containing a produced diaryl carbonate from a lower portion of said second column in a liquid form, and continuously feeding the second column low boiling point reaction mixture containing the dialkyl carbonate into the first continuous multi-stage distillation column;

(II) purifying said diaryl carbonate so as to obtain a high-purity diaryl carbonate;

(III) reacting said aromatic dihydroxy compound and said high-purity diaryl carbonate together so as to produce an aromatic polycarbonate molten prepolymer, and producing an aromatic polycarbonate using a guide-contacting downflow type polymerization apparatus in which said molten prepolymer is made to flow down along surfaces of guides, and said molten prepolymer is polymerized while flowing down; and

(IV) circulating the aromatic monohydroxy compound by-produced in step (III) back into the diaryl carbonate production step (I) so as to recycle the aromatic monohydroxy compound;

wherein:

(a) said first continuous multi-stage distillation column comprises a structure having a cylindrical trunk portion having a length $L_1$ (cm) and an inside diameter $D_1$ (cm), and having an internal with number of stages $n_1$ thereinside, and has a gas outlet having an inside diameter $d_{11}$ (cm) at a top of the column or in an upper portion of the column near to the top, a liquid outlet having an inside diameter $d_{12}$ (cm) at a bottom of the column or in a lower portion of the column near to the bottom, at least one first inlet provided in the upper portion and / or a middle portion of the column below said gas outlet, and at least one second inlet provided in the middle portion and / or the lower portion of the column above said liquid outlet, wherein $L_1$, $D_1$, $L_1$ / $D_1$, $n_1$, $D_1$ / $d_{11}$, and $D_1$ / $d_{12}$ respectively satisfy the following formulae (1) to (6):

$$1500 \leq L_1 \leq 8000 \quad (1),$$

$$100 \leq D_1 \leq 2000 \quad (2),$$

$$2 \leq L_1 / D_1 \leq 40 \quad (3),$$

$$20 \leq n_1 \leq 120 \quad (4),$$

$$5 \leq D_1 / d_{11} \leq 30 \quad (5),$$

$$3 \leq D_1 / d_{12} \leq 20 \qquad (6);$$

and

(b) said second continuous multi-stage distillation column comprises a structure having a cylindrical trunk portion having a length $L_2$ (cm) and an inside diameter $D_2$ (cm), and having an internal with number of stages $n_2$ thereinside, and has a gas outlet having an inside diameter $d_{21}$ (cm) at a top of the column or in an upper portion of the column near to the top, a liquid outlet having an inside diameter $d_{22}$ (cm) at a bottom of the column or in an lower portion of the column near to the bottom, at least one third inlet provided in the upper portion and / or a middle portion of the column below said gas outlet, and at least one fourth inlet provided in the middle portion and / or the lower portion of the column above the liquid outlet, wherein $L_2$, $D_2$, $L_2$ / $D_2$, $n_2$, $D_2$ / $d_{21}$, and $D_2$ / $d_{22}$ respectively satisfy the following formulae (7) to (12):

$$1500 \leq L_2 \leq 8000 \qquad (7),$$

$$100 \leq D_2 \leq 2000 \qquad (8),$$

$$2 \leq L_2 / D_2 \leq 40 \qquad (9),$$

$$10 \leq n_2 \leq 80 \qquad (10),$$

$$2 \leq D_2 / d_{21} \leq 15 \qquad (11),$$

$$5 \leq D_2 / d_{22} \leq 30 \qquad (12);$$

and

(c) said guide-contacting downflow type polymerization apparatus comprises:

(1) an apparatus having a molten prepolymer receiving port, a perforated plate, a polymerization reaction zone having a plurality of guides that extend downward from said perforated plate provided in a space surrounded by said perforated plate, a side casing and a tapered bottom casing, a molten prepolymer feeding zone for feeding a molten prepolymer via said perforated plate onto the guides in the polymerization reaction zone, a vacuum vent provided in said polymerization reaction zone, an aromatic polycarbonate discharge port provided in a lowermost portion of the tapered bottom casing, and an aromatic polycarbonate discharge pump connected to said discharge port,

wherein:

(2) an internal sectional area A ($m^2$) taken through a horizontal plane of the side casing in said polymerization reaction zone satisfies the following formula (13),

$$0.7 \leq A \leq 300 \qquad (13),$$

(3) a ratio between said A ($m^2$) and an internal sectional area B ($m^2$) taken through a horizontal plane of the aromatic polycarbonate discharge port satisfies the following formula (14),

$$20 \leq A / B \leq 1000 \qquad (14),$$

(4) the bottom casing in said polymerization reaction zone is connected at an internal angle C (°) to the side casing thereabove, wherein said angle C (°) satisfies the following formula (15),

$$120 \leq C \leq 165 \qquad (15),$$

(5) a length h (cm) of said guides satisfies the following formula (16),

$$150 \leq h \leq 5000 \qquad (16),$$

and
(6) a total external surface area S ($m^2$) of said guides satisfies the following formula (17),

$$2 \leq S \leq 50000 \qquad (17),$$

2. the process according to item 1, wherein not less than 1 ton / hr of the aromatic polycarbonate is produced,
3. the process according to item 1 or 2, wherein said $d_{11}$ and said $d_{12}$ satisfy the following formula (18), and said $d_{21}$ and said $d_{22}$ satisfy the following formula (19),

$$1 \leq d_{12} / d_{11} \leq 5 \qquad (18)$$

$$1 \leq d_{21} / d_{22} \leq 6 \qquad (19),$$

4. the process according to any one of items 1 to 3, wherein $L_1$, $D_1$, $L_1 / D_1$, $n_1$, $D_1 / d_{11}$, and $D_1 / d_{12}$ for said first continuous multi-stage distillation column satisfy respectively $2000 \leq L_1 \leq 6000$, $150 \leq D_1 \leq 1000$, $3 \leq L_1 / D_1 \leq 30$, $30 \leq n_1 \leq 100$, $8 \leq D_1 / d_{11} \leq 25$, and $5 \leq D_1 / d_{12} \leq 18$, and $L_2$, $D_2$, $L_2 / D_2$, $n_2$, $D_2 / d_{21}$, and $D_2 / d_{22}$ for said second continuous multi-stage distillation column satisfy respectively $2000 \leq L_2 \leq 6000$, $150 \leq D_2 \leq 1000$, $3 \leq L_2 / D_2 \leq 30$, $15 \leq n_2 \leq 60$, $2.5 \leq D_2 / d_{21} \leq 12$, and $7 \leq D_2 / d_{22} \leq 25$,
5. the process according to any one of items 1 to 4, wherein $L_1$, $D_1$, $L_1 / D_1$, $n_1$, $D_1 / d_{11}$, and $D_1 / d_{12}$ for said first continuous multi-stage distillation column satisfy respectively $2500 \leq L_1 \leq 5000$, $200 \leq D_1 \leq 800$, $5 \leq L_1 / D_1 \leq 15$, $40 \leq n_1 \leq 90$, $10 \leq D_1 / d_{11} \leq 25$, and $7 \leq D_1 / d_{12} \leq 15$, and $L_2$, $D_2$, $L_2 / D_2$, $n_2$, $D_2 / d_{21}$, and $D_2 / d_{22}$ for said second continuous multi-stage distillation column satisfy respectively $2500 \leq L_2 \leq 5000$, $200 \leq D_2 \leq 800$, $5 \leq L_2 / D_2 \leq 15$, $20 \leq n_2 < 50$, $3 \leq D_2 / d_{21} \leq 10$, and $9 \leq D_2 / d_{22} \leq 20$,
6. the process according to any one of items 1 to 5, wherein each of said first continuous multi-stage distillation column and said second continuous multi-stage distillation column comprises a distillation column having a tray and / or a packing as said internal,
7. the process according to item 6, wherein said first continuous multi-stage distillation column comprises a plate type distillation column having a tray as said internal, and said second continuous multi-stage distillation column comprises a distillation column having both a packing and a tray as said internal,
8. the process according to item 6 or 7, wherein each of said trays in said first continuous multi-stage distillation column and said second continuous multi-stage distillation column is a sieve tray having a sieve portion and a downcomer portion,
9. the process according to item 8, wherein said sieve tray has 100 to 1000 holes / $m^2$ in said sieve portion,
10. the process according to item 8 or 9, wherein a cross-sectional area per one hole of said sieve tray is in a range of from 0.5 to 5 $cm^2$,

11. the process according to item 6 or 7, wherein said second continuous multi-stage distillation column comprises a distillation column having, as said internal, the packing in the upper portion of the column, and the tray in the lower portion of the column,

12. the process according to any one of items 6 to 11, wherein the packing as said internal in said second continuous multi-stage distillation column is one or a plurality of sets of structured packings,

13. the process according to item 12, wherein said structured packing in said second continuous multi-stage distillation column is at least one type selected from the group consisting of Mellapak, Gempak, Techno-pack, Flexipac, a Sulzer packing, a Goodroll packing, and Glitschgrid,

14. the process according to any one of items 1 to 13, wherein the diaryl carbonate purification step (II) is carried out by distillation,

15. the process according to any one of items 1 to 14, wherein the side casing in the polymerization reaction zone comprises a cylindrical portion with an inside diameter D (cm) and a length L (cm), the tapered bottom casing, which is connected to a lower portion of the side casing, is conical, and the discharge port, which is in a lowermost portion of said conical casing, is cylindrical with an inside diameter d (cm), wherein D, L and d satisfy the following formulae (20), (21), (22) and (23),

$$100 \leq D \leq 1800 \qquad (20),$$

$$5 \leq D / d \leq 50 \qquad (21),$$

$$0.5 \leq L / D \leq 30 \qquad (22),$$

and

$$h - 20 \leq L \leq h + 300 \qquad (23),$$

16. the process according to any one of items 1 to 15, wherein said h satisfies the following formula (24),

$$400 < h \leq 2500 \qquad (24),$$

17. the process according to any one of items 1 to 16, wherein one of said guides is cylindrical, or pipe-shaped and made to be such that the molten prepolymer cannot enter therein, with an outside diameter r (cm), wherein r satisfies the following formula (25),

$$0.1 \leq r \leq 1 \qquad (25),$$

18. the process according to any one of items 1 to 17, wherein the polymerization is carried out using a plurality of said guide-contacting downflow type polymerization apparatuses linked together,

19. the process according any one of items 1 to 18, wherein the plurality of the guide-contacting downflow type polymerization apparatuses according to Claim 18 comprise two polymerization apparatuses being a first guide-contacting downflow type polymerization apparatus and a second guide-contacting downflow type polymerization apparatus, wherein in a process in which a polymerization degree is increased in this order, a total external surface area S1 ($m^2$) of the guides in said first guide-contacting downflow type polymerization apparatus and a total external surface area S2 ($m^2$) of the guides in said second guide-contacting downflow type polymerization apparatus satisfy the following formula (26),

$$1 \leq S1 / S2 \leq 20 \quad (26),$$

20. a high-quality aromatic polycarbonate produced in an amount of not less than 1 ton / hr by the process according to any one of items 1 to 19.
21. the high-quality aromatic polycarbonate according to item 20, having a content of alkali metal and / or alkaline earth metal compounds in a range of from 0.1 to 0.01 ppm in terms of metallic elements therein, and a halogen content of not more than 1 ppb,
22. the high-quality aromatic polycarbonate according to item 20 or 21, being an aromatic polycarbonate having a main chain thereof partially branched through foreign linkages such as an ester linkage or a ether linkage, and having a content of the foreign linkage in a range of from 0.05 to 0.5 mol% based on carbonate linkage.

Advantageous Effect of Invention

According to the present invention, it has been discovered that, when producing the aromatic polycarbonate from the dialkyl carbonate and the aromatic dihydroxy compound, by carrying out the process according to the present invention which comprises the steps; (I) producing a diaryl carbonate using two reactive distillation columns each having a specified structure; (II) purifying the diaryl carbonate so as to obtain a high-purity diaryl carbonate; (III) subsequently producing an aromatic polycarbonate using a guide-contacting downflow type polymerization apparatus having a specified structure from a molten prepolymer obtained from the aromatic dihydroxy compound and the high-purity diaryl carbonate: and (IV) recycling by-produced aromatic monohydroxy compound into the step (I), a high-quality and high-performance aromatic polycarbonate having excellent mechanical properties and no discoloration can be produced on an industrial scale of not less than 1 ton I hr at a fast polymerization rate. Moreover, it has been discovered that a high-quality aromatic polycarbonate can be produced stably for a prolonged period of time of, for example, not less than 2000 hours, preferably not less than 3000 hours, more preferably not less than 5000 hours, with little variation in molecular weight. The present invention thus provides a process that achieves excellent effects as an industrial process for the production of a high-quality aromatic polycarbonate.

Brief Description of Drawings

FIG. 1 illustrates a schematic view of a first continuous reactive distillation column preferable for carrying out the present invention, the distillation column having internals provided in a trunk portion thereof;
FIG. 2 illustrate a schematic view of a second continuous reactive distillation column preferable for carrying out the present invention, the distillation column having provided, in a trunk portion thereof, internals comprising a structured packing in an upper portion and a sieve tray in a lower portion;
FIG. 3 illustrates a schematic view of an apparatus comprising the first continuous reactive distillation column and the second continuous reactive distillation column linked together preferable for carrying out the present invention;
FIG. 4 illustrates a schematic view of a guide-contacting downflow type polymerization apparatus preferable for carrying out the present invention; and
FIG. 5 illustrates a schematic view of a guide-contacting downflow type polymerization apparatus having a cylindrical side casing and a tapered bottom casing preferable for carrying out the present invention.

Description of Reference Numerals

(In Figures 1, 2 and 3)

1: gas outlet, 2: liquid outlet, 3 and 4: inlet, 5: end plate, $L_1$ and $L_2$: length of trunk portion, $D_1$ and $D_2$: inside diameter of trunk portion (cm), $d_{11}$ and $d_{21}$: inside diameter of gas outlet (cm), $d_{12}$ and $d_{22}$: inside diameter of liquid outlet (cm), 101: first continuous multi-stage distillation column, 201: second continuous multi-stage distillation column, 11, 12, and 21: inlet, 13 and 23: column top gas outlet, 14, 24, 18, and 28: heat exchanger, 17 and 27: column bottom liquid outlet, 16 and 26: column top component outlet, 31: column bottom component outlet of the second continuous multi-stage distillation column.

(In Figures 4 and 5)

1: molten prepolymer receiving port, 2: perforated plate, 3: molten prepolymer feeding zone, 4: guide, 5: polymerization reaction zone, 6: vacuum vent, 7: aromatic polycarbonate discharge port, 8: aromatic polycarbonate discharge pump, 9: inert gas feeding port which is used according to need, 10: side casing of the polymerization reaction zone, 11: tapered bottom casing of the polymerization reaction zone, 12: outlet of aromatic polycarbonate.

Best Mode for Carrying Out the Invention

In the following, the present invention is described in detail.

In the present invention, first, a step (I) of continuously producing a diaryl carbonate on an industrial scale from a dialkyl carbonate and an aromatic monohydroxy compound is carried out. The dialkyl carbonate used in step (I) is a compound represented by general formula (27);

$R^aOCOOR^a$ 27

wherein $R^a$ represents an alkyl group having 1 to 10 carbon atoms, an alicyclic group having 3 to 10 carbon atoms, or an aralkyl group having 6 to 10 carbon atoms. Examples of $R^a$ include alkyl groups such as methyl, ethyl, propyl (isomers), allyl, butyl (isomers), butenyl (isomers), pentyl (isomers), hexyl (isomers), heptyl (isomers), octyl (isomers), nonyl (isomers), decyl (isomers) and cyclohexylmethyl; alicyclic groups such as cyclopropyl, cyclobutyl, cyclopentyl, cyclohexyl and cycloheptyl; and aralkyl groups such as benzyl, phenethyl (isomers), phenylpropyl (isomers), phenylbutyl (isomers) and methylbenzyl (isomers). These alkyl groups, alicyclic groups and aralkyl groups may be substituted with other substituents such as lower alkyl groups, lower alkoxy groups, cyano groups or halogen atoms, and may also contain unsaturated bonds.

Examples of dialkyl carbonates having such an $R^a$ include dimethyl carbonate, diethyl carbonate, dipropyl carbonate (isomers), diallyl carbonate, dibutenyl carbonate (isomers), dibutyl carbonate (isomers), dipentyl carbonate (isomers), dihexyl carbonate (isomers), diheptyl carbonate (isomers), dioctyl carbonate (isomers), dinonyl carbonate (isomers), didecyl carbonate (isomers), dicyclopentyl carbonate, dicyclohexyl carbonate, dicycloheptyl carbonate, dibenzyl carbonate, diphenethyl carbonate (isomers), di(phenylpropyl) carbonate (isomers), di(phenylbutyl) carbonate (isomers), di(chlorobenzyl) carbonate (isomers), di(methoxybenzyl) carbonate (isomers), di(methoxymethyl) carbonate, di (methoxyethyl) carbonate (isomers), di(chloroethyl) carbonate (isomers) and di(cyanoethyl) carbonate (isomers).

Of these dialkyl carbonates, ones preferably used in the present invention are dialkyl carbonates in which $R^a$ is an alkyl group having not more than four carbon atoms and not containing a halogen atom. A particularly preferable one is dimethyl carbonate. Moreover, of preferable dialkyl carbonates, particularly preferable ones are dialkyl carbonates produced in a state substantially not containing a halogen, for example ones produced from an alkylene carbonate substantially not containing a halogen and an alcohol substantially not containing a halogen.

The aromatic monohydroxy compound used in step (I) is a compound represented by following general formula (28). The type of the aromatic monohydroxy compound is not limited, so long as the hydroxyl group is directly bonded to the aromatic group;

$Ar^3OH$ formula (28)

wherein $Ar^3$ represents an aromatic group having 5 to 30 carbon atoms. Examples of aromatic monohydroxy compounds having such an $Ar^3$ include phenol; various alkylphenols such as cresol (isomers), xylenol (isomers), trimethylphenol (isomers), tetramethylphenol (isomers), ethylphenol (isomers), propylphenol (isomers), butylphenol (isomers), diethylphenol (isomers), methylethylphenol (isomers), methylpropylphenol (isomers), dipropylphenol (isomers), methylbutylphenol (isomers), pentylphenol (isomers), hexylphenol (isomers) and cyclohexylphenol (isomers); various alkoxyphenols such as methoxyphenol (isomers) and ethoxyphenol (isomers); arylalkylphenols such as phenylpropylphenol (isomers); naphthol (isomers) and various substituted naphthols; and heteroaromatic monohydroxy compounds such as hydroxypyridine (isomers), hydroxycoumarin (isomers) and hydroxyquinoline (isomers).

One of these aromatic monohydroxy compounds may be used, or a mixture of a plurality may be used. Of these aromatic monohydroxy compounds, ones preferably used in the present invention are aromatic monohydroxy compounds in which $Ar^3$ is an aromatic group having 6 to 10 carbon atoms. Phenol is particularly preferable. Moreover, of these aromatic monohydroxy compounds, ones substantially not containing a halogen are preferably used in the present invention. The diaryl carbonate in the present invention is thus represented by following formula 1 as a general formula;

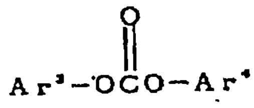

wherein each of $Ar^3$ and $Ar^4$ is a monovalent aromatic group.

Each of $Ar^3$ and $Ar^4$ represents a monovalent carbocyclic or heterocyclic aromatic group, and in each of $Ar^3$ and $Ar^4$, one or more of the hydrogen atoms may be substituted with another substituent that does not have an adverse effect on the reaction, for example a halogen atom, an alkyl group having 1 to 10 carbon atoms, an alkoxy group having 1 to 10 carbon atoms, a phenyl group, a phenoxy group, a vinyl group, a cyano group, an ester group, an amide group, a nitro group, or the like. $Ar^3$ and $Ar^4$ may be the same as one another, or different. Typical examples of each of the monovalent aromatic groups $Ar^3$ and $Ar^4$ include a phenyl group, a naphthyl group, a biphenyl group, and a pyridyl group. Each of these may be substituted with one or a plurality of substituent(s) as above. Preferable examples of each of $Ar^3$ and $Ar^4$ include those shown in following formula 2.

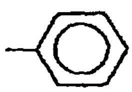
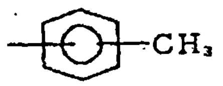
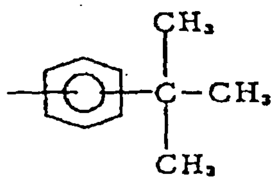

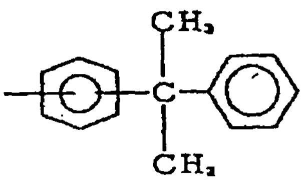
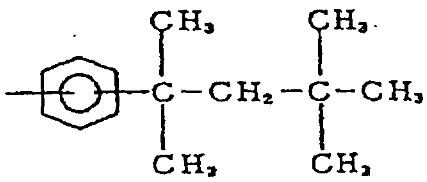

A particularly preferable diaryl carbonate is optionally substituted diphenyl carbonate represented by following formula 3:

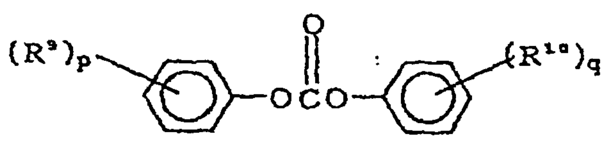

wherein each of $R^9$ and $R^{10}$ independently represents a hydrogen atom, an alkyl group having 1 to 10 carbon atoms, an alkoxy group having 1 to 10 carbon atoms, a cycloalkyl group having 5 to 10 carbon atoms in the ring thereof, or a phenyl group, and each of p and q is an integer from 1 to 5; in the case that p is 2 or more, the $R^9$'s may be different to one another, and in the case that q is 2 or more, the $R^{10}$'s may be different to one another.

Among these diaryl carbonates, a symmetrical diaryl carbonate such as unsubstituted diphenyl carbonate or a lower alkyl group-substituted diphenyl carbonate such as ditolyl carbonate or di-t-butylphenyl carbonate is preferable, diphenyl carbonate, which has the simplest structure, being particularly preferable. One such diaryl carbonate may be used alone, or a plurality may be used in combination.

A molar ratio of the dialkyl carbonate to the aromatic monohydroxy compound used in the starting material in step (I) is preferably in a range of from 0.1 to 10. Outside this range, an amount of unreacted starting material remaining based on a predetermined amount of the desired diaryl carbonate produced becomes high, which is not efficient, and moreover much energy is required to recover the starting material. For such reasons, the above molar ratio is more preferably in a range of from 0.5 to 5, yet more preferably from 0.8 to 3, still more preferably from 1 to 2.

In the present invention, not less than 1 ton / hr of the aromatic polycarbonate is continuously produced, and

for this, not less than approximately 0.85 ton / hr of high-purity diaryl carbonate must be continuously produced. In step (I), the minimum amount of the aromatic monohydroxy compound fed in continuously is thus generally 15 P ton I hr, preferably 13 P ton / hr, more preferably 10 P ton/hr, based on the amount of the aromatic polycarbonate (P ton / hr) to be produced. In a more preferable case, this amount can be made to be less than 8 P ton / hr.

The dialkyl carbonate and the aromatic monohydroxy compound used as the starting material in step (I) may each be of high purity, or may contain other compounds, for example may contain compounds or reaction by-products produced in the first continuous multi-stage distillation column and / or the second continuous multi-stage distillation column. In the case of industrial implementation, for the starting material, besides fresh dialkyl carbonate and aromatic monohydroxy compound newly introduced into the reaction system, it is also preferable to use dialkyl carbonate and / or aromatic monohydroxy compound recovered from the first continuous multi-stage distillation column and / or the second continuous multi-stage distillation column. In the process of the present invention, column top components constituting a low boiling point reaction mixture from the second continuous multi-stage distillation column are fed into the first continuous multi-stage distillation column. In this case, the second column low boiling point reaction mixture may be fed into the first continuous multi-stage distillation column as is, or after some of the components thereof have been separated out.

Accordingly, in the present invention, which is implemented industrially, it is preferable for the starting material fed into the first continuous multi-stage distillation column to contain any of an alcohol, an alkyl aryl carbonate, the diaryl carbonate, an alkyl aryl ether, and so on. The starting material further containing small amounts of high boiling point by-products such as Fries rearrangement products of the alkyl aryl carbonate and diaryl carbonate which are the products, derivatives thereof, and so on can also be preferably used. In the present invention, in the case, for example, of producing methylphenyl carbonate and diphenyl carbonate using a starting material containing dimethyl carbonate as the dialkyl carbonate and phenol as the aromatic monohydroxy compound, it is preferable for this starting material to contain methanol, and methyl phenyl carbonate and diphenyl carbonate, which are the reaction products, and the starting material may further contain small amounts of anisole, phenyl salicylate and methyl salicylate, which are reaction by-products, and high boiling point by-products derived therefrom.

Furthermore, most of the aromatic monohydroxy compound used in step (I) contain an aromatic monohydroxy compound by-produced in step (III) of the present invention. This by-produced aromatic monohydroxy compound must be circulated back into step (I) through step (IV).

The diaryl carbonate produced in step (I) is obtained through transesterification reaction between the dialkyl carbonate and the aromatic monohydroxy compound. Included under this transesterification are a reaction in which one or both of the alkoxy groups of the dialkyl carbonate is / are exchanged with the aryloxy group of the aromatic monohydroxy compound and an alcohol is eliminated, and a reaction in which two molecules of the alkyl aryl carbonate produced are converted into the diaryl carbonate and the dialkyl carbonate through a transesterification reaction therebetween, i.e. a disproportionation reaction. In step (I), in the first continuous multi-stage distillation column, the alkyl aryl carbonate is mainly obtained, and in the second continuous multi-stage distillation column, the diaryl carbonate and the dialkyl carbonate are mainly obtained through the disproportionation reaction of the alkyl aryl carbonate. The diaryl carbonate obtained in step (I) does not contain a halogen at all, and hence is important as a starting material when industrially producing the aromatic polycarbonate in the present invention. The reason for this is that if a halogen is present in the starting material for the polymerization even in a small amount of less than 1 ppm, then this may impede the polymerization reaction, thus impeding stable production of the aromatic polycarbonate, or cause a deterioration of the properties of, or discoloration of, the aromatic polycarbonate produced.

As a catalyst used in the first continuous multi-stage distillation column and / or the second continuous multi-stage distillation column in step (I), for example, a metal-containing compound selected from the following compounds can be used:

<Lead compounds>

lead oxides such as PbO, $PbO_2$ and $Pb_3O_4$;
lead sulfides such as PbS and $Pb_2S$;
lead hydroxides such as $Pb(OH)_2$ and $Pb_2O_2(OH)_2$;
plumbites such as $Na_2PbO_2$, $K_2PbO_2$, $NaHPbO_2$ and $KHPbO_2$;
plumbates such as $Na_2PbO_3$, $Na_2H_2PbO_4$, $K_2PbO_3$, $K_2[Pb(OH)_6]$, $K_4PbO_4$, $Ca_2PbO_4$ and $CaPbO_3$;
lead carbonates and basic salts thereof such as $PbCO_3$ and $2PbCO_3 \cdot Pb(OH)_2$;
lead salts of organic acids, and carbonates and basic salts thereof, such as $Pb(OCOCH_3)_2$, $Pb(OCOCH_3)_4$ and $Pb(OCOCH_3)_2 \cdot PbO \cdot 3H_2O$,
organolead compounds such as $Bu_4Pb$, $Ph_4Pb$, $Bu_3PbCl$, $Ph_3PbBr$, $Ph_3Pb$ (or $Ph_6Pb_2$), $Bu_3PbOH$ and $Ph_3PbO$

(wherein Bu represents a butyl group, and Ph represents a phenyl group);
alkoxylead compounds and aryloxylead compounds such as $Pb(OCH_3)_2$, $(CH_3O)Pb(OPh)$ and $Pb(OPh)_2$; lead alloys such as Pb-Na, Pb-Ca, Pb-Ba, Pb-Sn and Pb-Sb;
lead minerals such as galena and zinc blende; and hydrates of such lead compounds;

<Copper family metal compounds>

salts and complexes of copper family metals such as CuCl, $CuCl_2$, CuBr, $CuBr_2$, CuI, $CuI_2$, $Cu(OAc)_2$, $Cu(acac)_2$, copper oleate, $Bu_2Cu$, $(GH_3O)_2Cu$, $AgNO_3$, AgBr, silver picrate, $AgC_6H_6ClO_4$, $[AuC{\equiv}C\text{-}C(CH_3)_3]_n$ and $[Cu(C_7H_8)Cl]_4$ (wherein acac represents an acetylacetone chelate ligand);

<Alkali metal complexes>

alkali metal complexes such as Li(acac) and $LiN(C_4H_9)_2$;

<Zinc complexes>

zinc complexes such as $Zn(acac)_2$;

<Cadmium complexes>

cadmium complexes such as $Cd(acac)_2$;

<Iron family metal compounds>

complexes of iron family metals such as $Fe(C_{10}H_8)(CO)_5$, $Fe(CO)_5$, $Fe(C_4H_6)(CO)_3$, $Co(mesitylene)_2$, $(PEt_2Ph)_2$, $CoC_5F_5(CO)_7$, Ni-π-$C_5H_5NO$ and ferrocene;

<Zirconium complexes>

zirconium complexes such as $Zr(acac)_4$ and zirconocene;

<Lewis acid type compounds>

lewis acids and Lewis acid-forming transition metal compounds such as $AlX_3$, $TiX_3$, $TiX_4$, $VOX_3$, $VX_5$, $ZnX_2$, $FeX_3$ and $SnX_4$ (wherein X represents a halogen atom, an acetoxy group, an alkoxy group or an aryloxy group); and

<Organo-tin compounds>

organo-tin compounds such as $(CH_3)_3SnOCOCH_3$, $(C_2H_5)_3SnOCOC_6H_5$, $B_3SnOCOCH_3$, $Ph_3SnOCOCH_3$, $Bu_2Sn(OCOCH_3)_2$, $Bu_2Sn(OCOC_{11}H_{23})_2$, $Ph_3SnOCH_3$, $(C_2H_5)_3SnOPh$, $Bu_2Sn(OCH_3)_2$, $Bu_2Sn(OC_2H_5)_2$, $Bu_2Sn(OPh)_2$, $Ph_2Sn(OCH_3)_2$, $(C_2H_5)_3SnOH$, $Ph_3SnOH$, $Bu_2SnO$, $(C_8H_{17})_2SnO$, $Bu_2SnCl_2$ and BuSnO(OH). Each of these catalysts may be a solid catalyst fixed inside the multi-stage distillation column, or may be a soluble catalyst that dissolves in the reaction system.

Each of these catalyst components may of course have been reacted with an organic compound present in the reaction system such as an aliphatic alcohol, the aromatic monohydroxy compound, the alkyl aryl carbonate, the diaryl carbonate or the dialkyl carbonate, or may have been subjected to heating treatment with the starting material or products prior to the reaction.

In the case of carrying out step (I) with a soluble catalyst that dissolves in the reaction system, the catalyst is preferably one having a high solubility in the reaction liquid under the reaction conditions. Examples of preferable catalysts in this sense include PbO, $Pb(OH)_2$ and $Pb(OPh)_2$; $TiCl_4$, $Ti(OMe)_4$, $(MeO)Ti(OPh)_3$, $(MeO)_2Ti(OPh)_2$, $(MeO)_3Ti(OPh)$ and $Ti(OPh)_4$; $SnCl_4$, $Sn(OPh)_4$, $Bu_2SnO$ and $Bu_2Sn(OPh)_2$; $FeCl_3$, $Fe(OH)_3$ and $Fe(OPh)_3$; and such catalysts that have been treated with phenol, the reaction liquid or the like. The catalyst used in the first continuous multi-stage distillation column and the catalyst used in the second continuous multi-stage distillation column may be the same as one another, or different.

The first continuous multi-stage distillation column used in step (I) comprises a structure having a cylindrical trunk portion having a length $L_1$ (cm) and an inside diameter $D_1$ (cm), and having an internal with number of stages $n_1$ thereinside, and has a gas outlet having an inside diameter $d_{11}$ (cm) at a top of the column or in an upper portion of the column near to the top, a liquid outlet having an inside diameter $d_{12}$ (cm) at a bottom of the column or in a lower portion of the column near to the bottom, at least one first inlet provided in the upper portion and / or a middle portion of the column below the gas outlet, and at least one second inlet provided in the middle portion and / or the lower portion of the column above the liquid outlet, wherein $L_1$, $D_1$, $L_1 / D_1$, $n_1$, $D_1 / d_{11}$, and $D_1 / d_{12}$ must satisfy the following formulae (1) to (6) respectively:

$$1500 \leq L_1 \leq 8000 \quad (1),$$

$$100 \leq D_1 \leq 2000 \quad (2),$$

$$2 \leq L_1 / D_1 \leq 40 \quad (3),$$

$$20 \leq n_1 \leq 120 \quad (4),$$

$$5 \leq D_1 / d_{11} \leq 30 \quad (5),$$

and

$$3 \leq D_1 / d_{12} \leq 20 \quad (6).$$

Moreover, the second continuous multi-stage distillation column used in step (I) comprises a structure having a cylindrical trunk portion having a length $L_2$ (cm) and an inside diameter $D_2$ (cm), and having an internal with number of stages $n_2$ thereinside, and has a gas outlet having an inside diameter $d_{21}$ (cm) at a top of the column or in an upper portion of the column near to the top, a liquid outlet having an inside diameter $d_{22}$ (cm) at a bottom of the column or in a lower portion of the column near to the bottom, at least one third inlet provided in the upper portion and / or a middle portion of the column below the gas outlet, and at least one fourth inlet provided in the middle portion and / or the lower portion of the column above the liquid outlet, wherein $L_2$, $D_2$, $L_2 / D_2$, $n_2$, $D_2 / d_{21}$, and $D_2 / d_{22}$ must satisfy the following formulae (7) to (12) respectively:

$$1500 \leq L_2 \leq 8000 \quad (7),$$

$$100 \leq D_2 \leq 2000 \quad (8),$$

$$2 \leq L_2 / D_2 \leq 40 \quad (9),$$

$$10 \leq n_2 \leq 80 \quad (10),$$

$$2 \leq D_2 / d_{21} \leq 15 \quad (11),$$

and

$$5 \leq D_2 / d_{22} \leq 30 \quad (12).$$

It has been discovered that by using the first continuous multi-stage distillation column and the second continuous multi-stage distillation column that simultaneously satisfy the above formulae (1) to (12), the diaryl carbonate can be produced from the dialkyl carbonate and the aromatic monohydroxy compound on an industrial scale of not less than approximately 0.85 ton / hr, preferably not less than 1 ton / hr, with high selectivity and high productivity stably for a prolonged period of time, for example not less than 2000 hours, preferably not less than 3000 hours, more preferably not less than 5000 hours. The reason why it has become possible to produce such an aromatic carbonate on an industrial scale with such excellent effects by implementing the process of the present invention is not clear, but this is supposed to be due to a composite effect brought about when the conditions of the formulae (1) to (12) are combined. Preferable ranges for the respective factors for the continuous multi-stage distillation columns used in step (I) are described below.

If $L_1$ (cm) or $L_2$ (cm) is less than 1500, then the conversion decreases and hence it is not possible to attain the desired production amount. Moreover, to keep down the equipment cost while securing the conversion enabling the desired production amount to be attained, $L_1$ and $L_2$ must each be made to be not more than 8000. More preferable ranges for $L_1$ (cm) and $L_2$ (cm) are respectively $2000 \leq L_1 \leq 6000$ and $2000 \leq L_2 \leq 6000$, with $2500 \leq L_1 \leq 5000$ and $2500 \leq L_2 \leq 5000$ being yet more preferable.

If $D_1$ (cm) or $D_2$ (cm) is less than 100, then it is not possible to attain the desired production amount. Moreover, to keep down the equipment cost while attaining the desired production amount, $D_1$ and $D_2$ must each be made to be less than 2000. More preferable ranges for $D_1$ (cm) and $D_2$ (cm) are respectively $150 \leq D_1 \leq 1000$ and $150 \leq D_2 \leq 1000$, with $200 \leq D_1 \leq 800$ and $200 \leq D_2 \leq 800$ being yet more preferable. Note that for the first continuous multi-stage distillation column and the second continuous multi-stage distillation column, so long as $D_1$ and $D_2$ are within the above ranges, each of the columns may have the same inside diameter from the upper portion thereof to the lower portion thereof, or the inside diameter may differ for different portions. For example, for each of the continuous multi-stage distillation columns, the inside diameter of the upper portion of the column may be smaller than, or larger than, the inside diameter of the lower portion of the column.

If $L_1 / D_1$ or $L_2 / D_2$ is less than 2 or greater than 40, then stable operation becomes difficult. In particular, if $L_1 / D_1$ or $L_2 / D_2$ is greater than 40, then the pressure difference between the top and bottom of the column becomes too great, and hence prolonged stable operation becomes difficult. Moreover, it becomes necessary to increase the temperature in the lower portion of the column, and hence side reactions become liable to occur, bringing about a decrease in the selectivity. More preferable ranges for $L_1 / D_1$ and $L_2 / D_2$ are respectively $3 \leq L_1 / D_1 \leq 30$ and $3 \leq L_2 / D_2 \leq 30$, with $5 \leq L_1 / D_1 \leq 15$ and $5 \leq L_2 / D_2 \leq 15$ being yet more preferable.

If $n_1$ is less than 20, then the conversion decreases and it is not possible to attain the desired production amount for the first continuous multi-stage distillation column. Moreover, to keep down the equipment cost while securing the conversion enabling the desired production amount to be attained, $n_1$ must be made to be not more than 120. Furthermore, if $n_1$ is greater than 120, then the pressure difference between the top and bottom of the column becomes too great, and hence prolonged stable operation of the first continuous multi-stage distillation column becomes difficult. Moreover, it becomes necessary to increase the temperature in the lower portion of the column, and hence side reactions become liable to occur, bringing about a decrease in the selectivity. A more preferable range for $n_1$ is $30 \leq n_1 \leq 100$, with $40 \leq$

$n_1 \leq 90$ being yet more preferable.

Moreover, if $n_2$ is less than 10, then the conversion decreases and it is not possible to attain the desired production amount for the second continuous multi-stage distillation column. Moreover, to keep down the equipment cost while securing the conversion enabling the desired production amount to be attained, $n_2$ must be made to be not more than 80. Furthermore, if $n_2$ is greater than 80, then the pressure difference between the top and bottom of the column becomes too great, and hence prolonged stable operation of the second continuous multi-stage distillation column becomes difficult. Moreover, it becomes necessary to increase the temperature in the lower portion of the column, and hence side reactions become liable to occur, bringing about a decrease in the selectivity. A more preferable range for $n_2$ is $15 \leq n_2 \leq 60$, with $20 \leq n_2 \leq 50$ being yet more preferable.

If $D_1 / d_{11}$ is less than 5, then the equipment cost for the first continuous multi-stage distillation column becomes high. Moreover, large amounts of gaseous components are readily released to the outside of the system, and hence stable operation of the first continuous multi-stage distillation column becomes difficult. If $D_1 / d_{11}$ is greater than 30, then the gaseous component withdrawal amount becomes relatively low, and hence stable operation becomes difficult, and moreover a decrease in the conversion is brought about. A more preferable range for $D_1 / d_{11}$ is $8 \leq D_1 / d_{11} \leq 25$, with $10 \leq D_1 / d_{11} \leq 20$ being yet more preferable. Furthermore, if $D_2 / d_{21}$ is less than 2, then the equipment cost for the second continuous multi-stage distillation column becomes high. Moreover, large amounts of gaseous components are readily released to the outside of the system, and hence stable operation of the second continuous multi-stage distillation column becomes difficult. If $D_2 / d_{21}$ is greater than 15, then the gaseous component withdrawal amount becomes relatively low, and hence stable operation becomes difficult, and moreover a decrease in the conversion is brought about. A more preferable range for $D_2 / d_{21}$ is $5 \leq D_2 / d_{21} \leq 12$, with $3 \leq D_2 / d_{21} \leq 10$ being yet more preferable.

If $D_1 / d_{12}$ is less than 3, then the equipment cost for the first continuous multi-stage distillation column becomes high. Moreover, the liquid withdrawal amount becomes relatively high, and hence stable operation of the first continuous multi-stage distillation column becomes difficult. If $D_1 / d_{12}$ is greater than 20, then the flow rate through the liquid outlet and piping becomes excessively fast, and hence erosion becomes liable to occur, bringing about corrosion of the apparatus. A more preferable range for $D_1 / d_{12}$ is $5 \leq D_1 / d_{12} \leq 18$, with $7 \leq D_1 / d_{12} \leq 15$ being yet more preferable. Furthermore, if $D_2 / d_{22}$ is less than 5, then the equipment cost for the second continuous multi-stage distillation column becomes high. Moreover, the liquid withdrawal amount becomes relatively high, and hence stable operation of the second continuous multi-stage distillation column becomes difficult. If $D_2 / d_{22}$ is greater than 30, then the flow rate through the liquid outlet and piping becomes excessively fast, and hence erosion becomes liable to occur, bringing about corrosion of the apparatus. A more preferable range for $D_2 / d_{22}$ is $7 \leq D_2 / d_{22} \leq 25$, with $9 \leq D_2 / d_{22} \leq 20$ being yet more preferable.

Furthermore, it has been found that in step (I) it is further preferable for $d_{11}$ and $d_{12}$ to satisfy the following formula (18), and for $d_{21}$ and $d_{22}$ to satisfy the following formula (19).

$$1 \leq d_{12} / d_{11} \leq 5 \qquad (18),$$

and

$$1 \leq d_{21} / d_{22} \leq 6 \qquad (19).$$

The term "prolonged stable operation" for step (I) means that operation can be carried out continuously in a steady state based on the operating conditions with no flooding, clogging of piping, erosion or the like for not less than 1000 hours, preferably not less than 3000 hours, more preferably not less than 5000 hours, and a predetermined amount of the diaryl carbonate can be produced while maintaining high selectivity.

A characteristic feature for step (I) is that the diaryl carbonate can be produced stably for a prolonged period of time with high selectivity and with a high productivity of not less than 1 ton / hr, preferably not less than 2 ton / hr, more preferably not less than 3 ton / hr. Moreover, another characteristic feature for step (I) is that in the case that $L_1$, $D_1$, $L_1 / D_1$, $n_1$, $D_1 / d_{11}$, and $D_1 / d_{12}$ for the first continuous multi-stage distillation column satisfy respectively $2000 \leq L_1 \leq 6000$, $150 \leq D_1 \leq 1000$, $3 \leq L_1 / D_1 \leq 30$, $30 \leq n_1 \leq 100$, $8 \leq D_1 / d_{11} \leq 25$, and $5 \leq D_1 / d_{12} \leq 18$, and $L_2$, $D_2$, $L_2 / D_2$, $n_2$, $D_2 / d_{21}$, and $D_2 / d_{22}$ for the second continuous multi-stage distillation column satisfy respectively $2000 \leq L_2 \leq 6000$, $150 \leq D_2 \leq 1000$, $3 \leq L_2 / D_2 \leq 30$, $15 \leq n_2 \leq 60$, $2.5 \leq D_2 / d_{21} \leq 12$, and $7 \leq D_2 / d_{22} \leq 25$, not less than 2 ton / hr, preferably not less than 2.5 ton / hr, more preferably not less than 3 ton / hr of the diaryl carbonate can be produced.

Furthermore, another characteristic feature for step (I) is that in the case that $L_1$, $D_1$, $L_1 / D_1$, $n_1$, $D_1 / d_{11}$, and $D_1 / d_{12}$ for the first continuous multi-stage distillation column satisfy respectively $2500 \leq L_1 \leq 5000$, $200 \leq D_1 \leq 800$, 5

$\leq L_1 / D_1 \leq 15$, $40 \leq n_1 \leq 90$, $10 \leq D_1 / d_{11} \leq 25$, and $7 \leq D_1 / d_{12} \leq 15$, and $L_2$, $D_2$, $L_2 / D_2$, $n_2$, $D_2 / d_{21}$, and $D_2 / d_{22}$ for the second continuous multi-stage distillation column satisfy respectively $2500 \leq L_2 \leq 5000$, $200 \leq D_2 \leq 800$, $5 \leq L_2 / D_2 \leq 10$, $20 \leq n_2 \leq 50$, $3 \leq D_2 / d_{21} \leq 10$, and $9 \leq D_2 / d_{22} \leq 20$, not less than 3 ton / hr, preferably not less than 3.5 ton / hr, more preferably not less than 4 ton / hr of the diaryl carbonate can be produced.

The "selectivity" for the diaryl carbonate in step (I) is based on the aromatic monohydroxy compound reacted. In step (I), a high selectivity of not less than 95% can generally be attained, preferably not less than 97%, more preferably not less than 98%.

Each of the first continuous multi-stage distillation column and the second continuous multi-stage distillation column used in step (I) preferably comprises a distillation column having a tray and / or a packing as the internal. The term "internal" used in the present invention means the parts in the distillation column where gas and liquid are actually brought into contact with one another. Preferable examples of the tray include a bubble-cap tray, a sieve tray, a valve tray, a counterflow tray, a Superfrac tray, a Maxfrac tray, or the like. Preferable examples of the packing include a random packing such as a Raschig ring, a Lessing ring, a Pall ring, a Berl saddle, a Intalox saddle, a Dixon packing, a McMahon packing or Heli-Pak, or a structured packing such as Mellapak, Gempak, Techno-pack, Flexipac, a Sulzer packing, a Goodroll packing or Glitschgrid. A multi-stage distillation column having both a tray portion and a portion packed with the packing can also be used. Furthermore, the term "number of stages n of the internal" used in the present invention means the number of the tray in the case of the tray, and the theoretical number of stages in the case of the packing. In the case of the multi-stage distillation column having both the tray portion and the portion packed with the packing, n is thus the sum of the number of tray and the theoretical number of stages.

In step (I) in the first continuous multi-stage distillation column, a reaction in which the alkyl aryl carbonate is produced from the dialkyl carbonate and the aromatic monohydroxy compound mainly occurs. This reaction has an extremely low equilibrium constant, and the reaction rate is slow, and hence it has been discovered that a plate type distillation column having the tray as the internal is particularly preferable as the first continuous multi-stage distillation column used in the reactive distillation. Moreover, in the second continuous multi-stage distillation column, it is mainly a disproportionation reaction of the alkyl aryl carbonate that occurs. This reaction also has a low equilibrium constant, and a slow reaction rate. Even so, it has been discovered that a distillation column having both the packing and the tray as the internal is preferable as the second continuous multi-stage distillation column used in the reactive distillation. Furthermore, it has been discovered that a distillation column having the packing installed in the upper portion thereof and the tray installed in the lower portion thereof is preferable as the second continuous multi-stage distillation column. It has been discovered that the packing in the second continuous multi-stage distillation column is preferably structured packing, and that of structured packing, Mellapak is particularly preferable.

Furthermore, it has been discovered that, as each of said tray installed in the first continuous multi-stage distillation column and the second continuous multi-stage distillation column, a sieve tray having a sieve portion and a downcomer portion is particularly good in terms of the relationship between performance and equipment cost. It has also been discovered that each sieve tray preferably has from 100 to 1000 holes / $m^2$ in the sieve portion. A more preferable number of holes is from 120 to 900 holes / $m^2$, yet more preferably from 150 to 800 holes / $m^2$.

Moreover, it has been discovered that the cross-sectional area per hole of each sieve tray is preferably in a range of from 0.5 to 5 $cm^2$. A more preferable cross-sectional area per hole is from 0.7 to 4 $cm^2$, yet more preferably from 0.9 to 3 $cm^2$. Furthermore, it has been discovered that it is particularly preferable if each sieve tray has from 100 to 1000 holes / $m^2$ in the sieve portion, and the cross-sectional area per hole is in a range of from 0.5 to 5 $cm^2$. It has been shown that by adding the above conditions to the continuous multi-stage distillation columns, the object of the present invention can be attained more easily.

When carrying out step (I), the diaryl carbonate is continuously produced by continuously feeding the dialkyl carbonate and the aromatic monohydroxy compound as the starting material into the first continuous multi-stage distillation column in which a catalyst is present, carrying out reaction and distillation simultaneously in the first column, continuously withdrawing a first column low boiling point reaction mixture containing a produced alcohol from an upper portion of the first column in a gaseous form, continuously withdrawing a first column high boiling point reaction mixture containing a produced alkyl aryl carbonate from a lower portion of the first column in a liquid form, continuously feeding the first column high boiling point reaction mixture into the second continuous multi-stage distillation column in which a catalyst is present, carrying out reaction and distillation simultaneously in the second column, continuously withdrawing a second column low boiling point reaction mixture containing a produced dialkyl carbonate from an upper portion of the second column in a gaseous form, continuously withdrawing a second column high boiling point reaction mixture containing a produced diaryl carbonate from a lower portion of the second column in a liquid form, and continuously feeding the second column low boiling point reaction mixture containing the dialkyl carbonate into the first continuous multi-stage distillation column. As mentioned earlier, the starting material may contain the alcohols, the alkyl aryl carbonate and the diaryl carbonate that are reaction products, and reaction by-products such as an alkyl aryl ether and high boiling point compounds. Considering the equipment and cost required for separation and purification in other processes, when actually implementing the present invention industrially, it is preferable for the starting material to contain small amounts

of such compounds.

In step (I), when continuously feeding the dialkyl carbonate and the aromatic monohydroxy compound as the starting material into the first continuous multi-stage distillation column, this starting material may be fed into the first distillation column in a liquid form and / or a gaseous form from inlet(s) provided in one or a plurality of positions in the upper portion or the middle portion of the first distillation column below the gas outlet in the upper portion of the first distillation column. It is also preferable to feed a starting material containing a large proportion of the aromatic monohydroxy compound into the first distillation column in a liquid form from an inlet provided in the upper portion of the first distillation column, and feed a starting material containing a large proportion of the dialkyl carbonate into the first distillation column in a gaseous form from an inlet provided in the lower portion of the first distillation column above the liquid outlet in the lower portion of the first distillation column.

Moreover, in step (I), the first column high boiling point reaction mixture containing the alkyl aryl carbonate continuously withdrawn from the lower portion of the first continuous multi-stage distillation column is continuously fed into the second continuous multi-stage distillation column. Here, the first column high boiling point reaction mixture is preferably fed into the second distillation column in a liquid form and / or a gaseous form from inlet(s) provided in one or a plurality of positions in the upper portion or the middle portion of the second distillation column below the gas outlet in the upper portion of the second distillation column. Moreover, in the case of using, as the second distillation column, a distillation column having the packing portion in the upper portion thereof and the tray portion in the lower portion thereof, which is a preferable embodiment of the present invention, it is preferable for at least one position where an inlet is provided to be between the packing portion and the tray portion. Moreover, in the case that the packings comprise a plurality of sets of structured packings, it is preferable for an inlet to be installed in a space between the sets of structured packings.

Moreover, in step (I), it is also preferable to carry out a reflux operation of condensing the gaseous component withdrawn from the top of each of the first continuous multi-stage distillation column and the second continuous multi-stage distillation column, and then returning some of this component into the upper portion of the respective distillation column. In this case, a reflux ratio for the first continuous multi-stage distillation column is in a range of from 0 to 10, and a reflux ratio for the second continuous multi-stage distillation column is in a range of from 0.01 to 10, preferably from 0.08 to 5, more preferably from 0.1 to 2. For the first continuous multi-stage distillation column, not carrying out such a reflux operation (i.e. reflux ratio = 0) is also a preferable embodiment.

In step (I), the method of making the catalyst be present in the first continuous multi-stage distillation column may be any method. In the case that the catalyst is a solid that is insoluble in the reaction liquid, it is preferable for the catalyst to be fixed inside the column by, for example, being installed on stages inside the first continuous multi-stage distillation column or being installed in the form of a packing. In the case of a catalyst that dissolves in the starting material or the reaction liquid, it is preferable to feed the catalyst into the first distillation column from a position above the middle portion of the first distillation column. In this case, a catalyst solution obtained by dissolving the catalyst in the starting material or reaction liquid may be introduced into the column together with the starting material, or may be introduced into the column from a different inlet to the starting material. The amount of the catalyst used in the first continuous multi-stage distillation column in the present invention varies depending on the type of catalyst used, the types and proportions of the starting material compounds, and reaction conditions such as the reaction temperature and the reaction pressure. The amount of the catalyst is generally in a range of from 0.0001 to 30 % by weight, preferably from 0.0005 to 10 % by weight, more preferably from 0.001 to 1 % by weight, based on the total weight of the starting material.

Moreover, in step (I), the method of making the catalyst be present in the second continuous multi-stage distillation column may be any method. In the case that the catalyst is a solid that is insoluble in the reaction liquid, it is preferable for the catalyst to be fixed inside the column by, for example, being installed on stages inside the second continuous multi-stage distillation column or being installed in the form of a packing. In the case of a catalyst that dissolves in the starting material or the reaction liquid, it is preferable to feed the catalyst into the second distillation column from a position above the middle portion of the second distillation column. In this case, a catalyst solution obtained by dissolving the catalyst in the starting material or reaction liquid may be introduced into the column together with the starting material, or may be introduced into the column from a different inlet to the starting material. The amount of the catalyst used in the second continuous multi-stage distillation column in the present invention varies depending on the type of catalyst used, the types and proportions of the starting material compounds, and reaction conditions such as the reaction temperature and the reaction pressure. The amount of the catalyst is generally in a range of from 0.0001 to 30 % by weight, preferably from 0.0005 to 10 % by weight, more preferably from 0.001 to 1 % by weight, based on the total mass of the starting material.

In step (I), the catalyst used in the first continuous multi-stage distillation column and the catalyst used in the second continuous multi-stage distillation column may be the same or different, but are preferably the same. More preferably, the same catalyst is used in both columns, this catalyst being one that dissolves in the reaction liquid in both columns. In this case, the catalyst dissolved in the high boiling point reaction mixture in the first continuous multi-stage distillation column is generally withdrawn from the lower portion of the first distillation column together with the alkyl aryl

carbonate and so on, and fed into the second continuous multi-stage distillation column as is; this is a preferable embodiment. Note that, if necessary, more of the catalyst can be newly added into the second continuous multi-stage distillation column.

The reaction times for the transesterification reactions carried out in step (I) are considered to equate to the average residence times of the reaction liquids in the first continuous multi-stage distillation column and the second continuous multi-stage distillation column. Each of these reaction times varies depending on the form of the internal in that distillation column and the number of stages, the amount of the starting material fed into the column, the type and amount of the catalyst, the reaction conditions, and so on. The reaction time in each of the first continuous multi-stage distillation column and the second continuous multi-stage distillation column is generally in a range of from 0.01 to 10 hours, preferably from 0.05 to 5 hours, more preferably from 0.1 to 3 hours.

The reaction temperature in the first continuous multi-stage distillation column varies depending on the type of the starting material compounds used, and the type and amount of the catalyst. This reaction temperature is generally in a range of from 100 to 350°C. It is preferable to increase the reaction temperature so as to increase the reaction rate. However, if the reaction temperature is too high, then side reactions become liable to occur, for example production of by-products such as an alkyl aryl ether increases, which is undesirable. For this reason, the reaction temperature in the first continuous multi-stage distillation column is preferably in a range of from 130 to 280°C, more preferably from 150 to 260°C, yet more preferably from 180 to 250°C.

The reaction temperature in the second continuous multi-stage distillation column varies depending on the type of the starting material compounds used, and the type and amount of the catalyst. This reaction temperature is generally in a range of from 100 to 350°C. It is preferable to increase the reaction temperature so as to increase the reaction rate. However, if the reaction temperature is too high, then side reactions become liable to occur, for example production of by-products such as an alkyl aryl ether, and Fries rearrangement products of the starting material compounds and the produced diaryl carbonate, and derivatives thereof increases, which is undesirable. For this reason, the reaction temperature in the second continuous multi-stage distillation column is preferably in a range of from 130 to 280°C, more preferably from 150 to 260°C, yet more preferably from 180 to 250°C.

Moreover, the reaction pressure in the first continuous multi-stage distillation column varies depending on the type of the starting material compounds used and the composition of the starting material, the reaction temperature, and so on. The first continuous multi-stage distillation column may be at any of a reduced pressure, normal pressure, or an applied pressure. The pressure at the top of the column is generally in a range of from 0.1 to $2\times10^7$ Pa, preferably from $10^5$ to $10^7$ Pa, more preferably from $2\times10^5$ to $5\times10^6$ Pa.

The reaction pressure in the second continuous multi-stage distillation column varies depending on the type of the starting material compounds used and the composition of the starting material, the reaction temperature, and so on. The second continuous multi-stage distillation column may be at any of a reduced pressure, normal pressure, or an applied pressure. The pressure at the top of the column is generally in a range of from 0.1 to $2\times10^7$ Pa, preferably from $10^3$ to $10^6$ Pa, more preferably from $5\times10^3$ to $10^5$ Pa.

As the first continuous multi-stage distillation column in step (I), a plurality of distillation columns may be used. In this case, the distillation columns may be linked together in series, in parallel, or in a combination of series and parallel. Moreover, as the second continuous multi-stage distillation column in step (I), a plurality of distillation columns may be used. In this case, the distillation columns may be linked together in series, in parallel, or in a combination of series and parallel.

The material constituting each of the first continuous multi-stage distillation column and the second continuous multi-stage distillation column used in step (I) is generally a metallic material such as carbon steel or stainless steel. In terms of the quality of the aromatic carbonates produced, stainless steel is preferable.

The second column high boiling point reaction mixture continuously withdrawn from the lower portion of the second continuous multi-stage distillation column in a liquid form in step (I) has the diaryl carbonate as a main component thereof, but generally also contains unreacted alkyl aryl carbonate, a small amount of unreacted starting material, a small amount of high boiling point by-products and so on, and moreover in the case that a homogeneous catalyst is used, also contains this catalyst component. It is thus necessary to carry out a purification step (II) for obtaining a high-purity diaryl carbonate from the second column high boiling point reaction mixture. In step (II), any process may be used, so long as the high-purity diaryl carbonate can be obtained from the second column high boiling point reaction mixture. Examples include distillation and / or recrystallization and so on. It has been discovered that, of these, in the present invention, it is particularly preferable to carry out step (II) using a distillation process.

Furthermore, in the present invention, it has been discovered that it is further preferable for step (II) to adopt a distillation separation process in which two distillation columns (a high boiling point material separating column, and a diaryl carbonate purifying column having a side cut outlet) are used, separation is carried out continuously in the high boiling point material separating column into a column top component having as a main component thereof the diaryl carbonate, unreacted alkyl aryl carbonate, and a small amount of unreacted starting material, and a column bottom component having as a main component thereof a small amount of high boiling point by-products and so on and / or a

catalyst component, the column top component from the high boiling point material separating column is continuously fed into the diaryl carbonate purifying column, and separation is carried out continuously in the diaryl carbonate purifying column into three components, i.e. a column top component, a side cut component, and a column bottom component, the high-purity diaryl carbonate being obtained as the side cut component.

Furthermore, it is preferable for all or some of the column bottom component from the high boiling point matter separating column to be circulated back into the first continuous multi-stage distillation column and / or the second continuous multi-stage distillation column and reused as catalyst component in step (I). Moreover, the column top component from the diaryl carbonate purifying column generally contains a small amount of the diaryl carbonate, and hence a method in which, with the column top component as is or after carrying out other distillation separation of the low boiling point components containing in said column top component using another column, some or all of said another column bottom component is returned into the high boiling point matter separating column and / or the diaryl carbonate purifying column, so as to be recovered as high-purity diaryl carbonate is also preferable.

In step (II), a high-purity diaryl carbonate of purity generally not less than 99.9%, preferably not less than 99.99%, is obtained. The content of high boiling point by-products therein is generally not more than 100 ppm, preferably not more than 50 ppm, more preferably not more than 10 ppm. Moreover, in the present invention, the starting material and catalyst not containing a halogen are generally used, and hence the halogen content of the high-purity diaryl carbonate obtained is not more than 0.1 ppm, preferably not more than 10 ppb, more preferably not more than 1 ppb.

Next, step (III) is carried out. This is a step of reacting an aromatic dihydroxy compound and the high-purity diaryl carbonate together so as to produce an aromatic polycarbonate molten prepolymer, and producing an aromatic polycarbonate using a guide-contacting downflow type polymerization apparatus in which the molten prepolymer is made to flow down along surfaces of guides, and the molten prepolymer is polymerized while flowing down.

The aromatic dihydroxy compound used in step (III) is a compound represented by general formula (29);

$$HO\text{-}Ar\text{-}OH \qquad (29)$$

wherein Ar represents a bivalent aromatic group.

The bivalent aromatic group Ar is preferably, for example, one represented by general formula (30);

$$\text{-}Ar^1\text{-}Y\text{-}Ar^2\text{-} \qquad (30)$$

wherein each of $Ar^1$ and $Ar^2$ independently represents a bivalent carbocyclic or heterocyclic aromatic group having 5 to 70 carbon atoms, and Y represents a bivalent alkane group having 1 to 30 carbon atoms.

In each of the bivalent aromatic groups $Ar^1$ and $Ar^2$, one or a plurality of the hydrogen atoms may be substituted with another substituent that does not have an adverse effect on the reaction, for example a halogen atom, an alkyl group having 1 to 10 carbon atoms, an alkoxy group having 1 to 10 carbon atoms, a phenyl group, a phenoxy group, a vinyl group, a cyano group, an ester group, an amide group, a nitro group, or the like. A preferable specific example of a heterocyclic aromatic group is an aromatic group having one or a plurality of ring-forming nitrogen atom(s), oxygen atom(s), and / or sulfur atom(s). Each of the bivalent aromatic groups $Ar^1$ and $Ar^2$ may represent, for example, a group such as optionally substituted phenylene, optionally substituted biphenylene, or optionally substituted pyridylene. Here, substituents are defined as above.

The bivalent alkane group Y is, for example, an organic group represented by following formula;

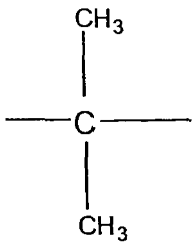 , 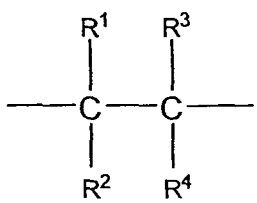 , 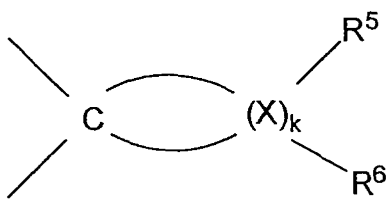

wherein each of $R^1$, $R^2$, $R^3$, and $R^4$ independently represents a hydrogen atom, an alkyl group having 1 to 10 carbon atoms, an alkoxy group having 1 to 10 carbon atoms, a cycloalkyl group having 5 to 10 carbon atoms in the ring thereof, a carbocyclic aromatic group having 5 to 10 carbon atoms in the ring thereof, or a carbocyclic aralkyl group having 6 to 10 carbon atoms, k represents an integer from 3 to 11, $R^5$ and $R^6$ are selected separately for each X, each independently

representing a hydrogen atom or an alkyl group having 1 to 6 carbon atoms, and each X represents a carbon atom. Moreover, in each of $R^1$, $R^2$, $R^3$, $R^4$, $R^5$, and $R^6$, one or more of the hydrogen atoms may be substituted with another substituent that does not have an adverse effect on the reaction, for example a halogen atom, an alkyl group having 1 to 10 carbon atoms, an alkoxy group having 1 to 10 carbon atoms, a phenyl group, a phenoxy group, a vinyl group, a cyano group, an ester group, an amide group, a nitro group, or the like.

Examples of such a bivalent aromatic group Ar are shown in following formula;

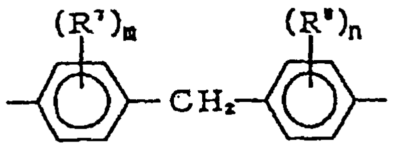
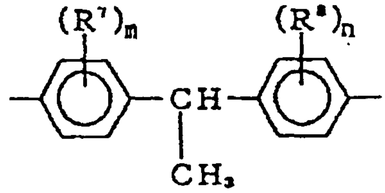
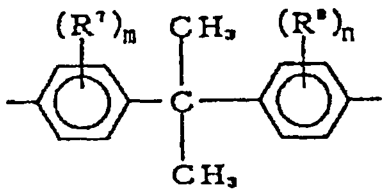
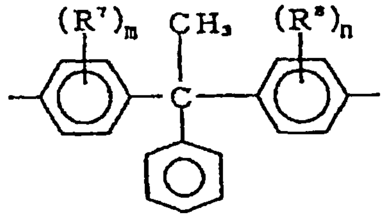
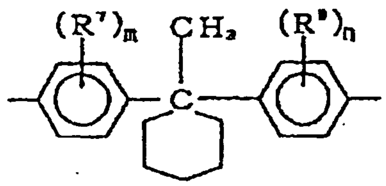
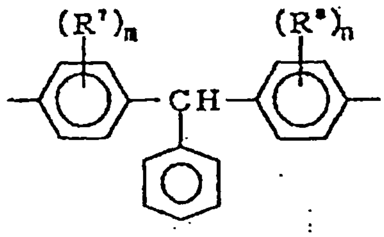
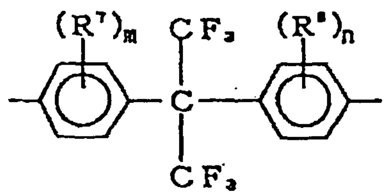
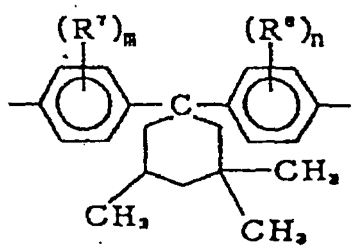
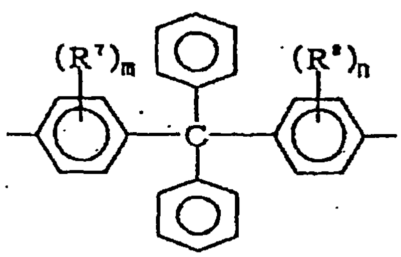
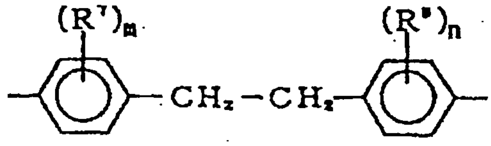

wherein each of $R^7$ and $R^8$ independently represents a hydrogen atom, a halogen atom, an alkyl group having 1 to 10 carbon atoms, an alkoxy group having 1 to 10 carbon atoms, a cycloalkyl group having 5 to 10 carbon atoms in the ring thereof, or a phenyl group, and each of m and n is an integer from 1 to 4; in the case that m is in a range of from 2 to 4, the $R^7$'s may be the same as or different to one another, and in the case that n is in a range of from 2 to 4, the $R^8$'s may be the same as or different to one another.

Furthermore, the bivalent aromatic group Ar may be one represented by the following formula;

$$-Ar^1-Z-Ar^2-$$

wherein $Ar^1$ and $Ar^2$ are defined as above, and Z is a single bond or a bivalent group such as -O-, -CO-, -S-, $-SO_2-$, -SO-, -COO-, or $-CON(R^1)-$, wherein $R^1$ is defined as above.

Examples of such a bivalent aromatic group Ar are shown in following formula;

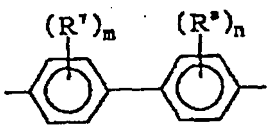

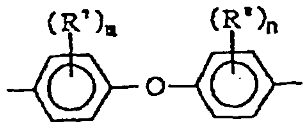

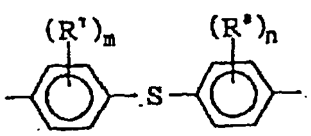

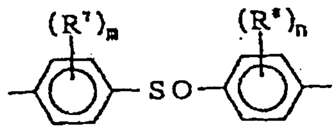

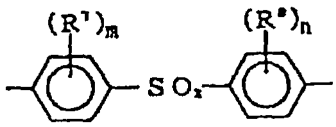

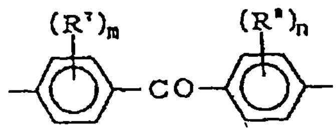

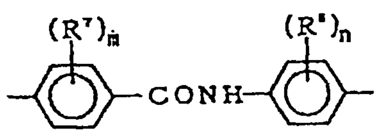

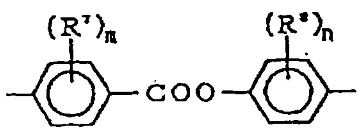

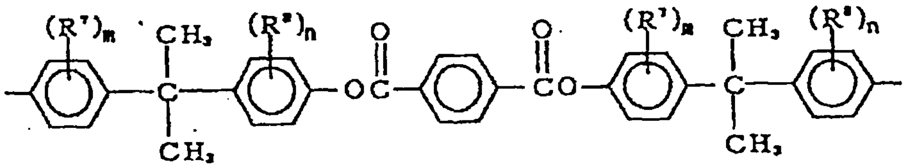

wherein $R^7$, $R^8$, m and n are defined as before.

Furthermore, specific examples of the bivalent aromatic group Ar include unsubstituted or substituted phenylene, unsubstituted or substituted naphthylene, and unsubstituted or substituted pyridylene.

In the present invention, one such aromatic dihydroxy compound may be used, or a plurality may be used. A typical example of the aromatic dihydroxy compound is bisphenol A. Moreover, in the present invention, the aromatic dihydroxy compound may be used together with a trivalent aromatic trihydroxy compound for introducing a branched structure so long as this is within a range such as not to impair the object of the present invention.

A ratio between the aromatic dihydroxy compound and the high-purity diaryl carbonate used in step (III) varies depending on the type of the aromatic dihydroxy compound and the diaryl carbonate which are used, and the polymerization temperature and other polymerization conditions, but the diaryl carbonate is generally used in a range of from 0.9 to 2.5 mol, preferably from 0.95 to 2.0 mol, more preferably from 0.98 to 1.5 mol, based on 1 mol of the aromatic dihydroxy compound.

The prepolymer in a molten state (hereinafter referred to as the "molten prepolymer") produced from the aromatic dihydroxy compound and the diaryl carbonate in step (III) means partially polymerized molten material produced from the aromatic dihydroxy compound and the diaryl carbonate having a lower polymerization degree than the aromatic polycarbonate having the desired polymerization degree, and may of course be an oligomer. The molten prepolymer used in step (III) may be obtained through any publicly known process. For example, the molten prepolymer can be produced by stirring a molten mixture containing predetermined amounts of the aromatic dihydroxy compound and the diaryl carbonate under normal pressure and / or a reduced pressure at a temperature in a range of from approximately 120 °C to approximately 280 °C using one or a plurality of the vertical stirring tank(s), while removing aromatic monohydroxy compound by-produced through the reaction. A process in which a plurality of the vertical stirring tanks linked together in series are used, and the polymerization degree is progressively increased so as to continuously produce the molten prepolymer having the required polymerization degree is particularly preferable.

In step (III), the molten prepolymer is continuously fed into a guide-contacting downflow type polymerization apparatus, so as to continuously produce an aromatic polycarbonate having a desired polymerization degree. The guide-contacting downflow type polymerization apparatus is a polymerization apparatus in which the prepolymer is subjected to polymerization while being made to fall down in a molten state along the guides, and is such that not less than 1 ton / hr of the aromatic polycarbonate can be produced.

The guide-contacting downflow type polymerization apparatus must be:

(1) an apparatus having a molten prepolymer receiving port, a perforated plate, a polymerization reaction zone having a plurality of guides that extend downward from the perforated plate provided in a space surrounded by the perforated plate, a side casing and a tapered bottom casing, a molten prepolymer feeding zone for feeding the molten prepolymer via the perforated plate onto the guides in the polymerization reaction zone, a vacuum vent provided in the polymerization reaction zone, an aromatic polycarbonate discharge port provided in a lowermost portion of the tapered bottom casing, and an aromatic polycarbonate discharge pump connected to the discharge port, wherein:

(2) an internal sectional area A ($m^2$) taken through a horizontal plane of the side casing in the polymerization reaction zone satisfies the following formula (13),

$$0.7 \leq A \leq 300 \qquad (13),$$

(3) a ratio between the A ($m^2$) and an internal sectional area B ($m^2$) taken through a horizontal plane of the aromatic polycarbonate discharge port satisfies the following formula (14),

$$20 \leq A / B \leq 1000 \qquad (14),$$

(4) the tapered bottom casing in the polymerization reaction zone is connected at an internal angle C (°) to the side casing thereabove, wherein the angle C (°) satisfies the following formula (15),

$$120 \leq C \leq 165 \qquad (15),$$

(5) a length h (cm) of the guides satisfies the following formula (16),

$$150 \leq h \leq 5000 \qquad (16),$$

and
(6) a total external surface area S ($m^2$) of the guides satisfies the following formula (17),

$$2 \leq S \leq 50000 \qquad (17).$$

To produce a high-quality high-performance aromatic polycarbonate stably for a prolonged period of time with no variation in molecular weight in an amount on an industrial scale of not less than 1 ton / hr, a polymerization apparatus satisfying various conditions is required; in the present invention, these conditions have been discovered. (In the present invention, "no variation in molecular weight" means that the variation in the number average molecular weight is not more than 200.) In the present invention, the aromatic polycarbonate can be produced stably for a prolonged period of time with variation in molecular weight being preferably no more than 150, more preferably not more than 100.

More specifically, the internal sectional area A ($m^2$) taken through a horizontal plane (plane a-a') of the side casing 10 in the polymerization reaction zone 5 as shown in the schematic view of FIG. 4 must satisfy the above formula (13).

If A is less than 0.7 $m^2$, then the desired production amount cannot be attained. Moreover, to attain such a production amount while keeping down the equipment cost, A must be made to be not more than 300 $m^2$.

Furthermore, the ratio between A ($m^2$) and the internal sectional area B ($m^2$) taken through a horizontal plane (plane b-b') of the aromatic polycarbonate discharge port 7 must satisfy the above formula (14).

A / B must satisfy the above formula (14) so that the molten matter of high melt viscosity containing the produced aromatic polycarbonate or aromatic polycarbonate prepolymer of increased polymerization degree can be discharged without causing a deterioration in the quality of this molten matter.

Furthermore, the tapered bottom casing 11 in the polymerization reaction zone 5 is provided at an internal angle C (°) to the side casing 10 thereabove, wherein the angle C (°) must satisfy the above formula (15).

To keep down the equipment cost, C is preferably as close as possible to 90°, but to move the molten matter of high melt viscosity containing the aromatic polycarbonate or aromatic polycarbonate prepolymer of increased polymerization degree falling down from the lower ends of guides 4 to the discharge port 7 without bringing about a reduction in the quality of this molten matter, C must satisfy the above formula (15).

Furthermore, the length h (cm) of each of the guides 4 must satisfy the above formula (16). It is undesirable for h to be shorter than 150 cm, since then the polymerization degree of the molten prepolymer cannot be increased sufficiently, and moreover the variation in the polymerization degree may become greater than approximately 200 in terms of the number average molecular weight. It is undesirable for h to be longer than 5000 cm, since then the difference in the melt viscosity of the molten prepolymer between the top and the bottom of each guide 4 becomes too great, and hence the variation in the polymerization degree becomes greater than approximately 300 (in some cases greater than approximately 500) in terms of the number average molecular weight, and hence variation arises in the properties of the aromatic polycarbonate obtained. Note that the variation in the polymerization degree being great in the present invention means, for example, the case of variation such that there is a difference of more than approximately 200 in terms of the number average molecular weight.

Furthermore, the total external surface area S ($m^2$) of the guides 4 must satisfy formula (16). If S is less than 2 $m^2$, then the desired production amount cannot be attained. Moreover, to attain such a production amount while keeping down the equipment cost, and to eliminate variation in properties, S must be made to be not more than 50000 $m^2$.

It has been discovered that by using the guide-contacting downflow type polymerization apparatus simultaneously satisfying the above formulae (13), (14), (15), (16) and (17), surprisingly, the high-quality high-performance aromatic polycarbonate having excellent mechanical properties and no discoloration can be produced in an amount of not less than 1 ton / hr for a prolonged period of time of not less than several thousand hours, for example not less than 5,000 hours, stably with no variation in the molecular weight. In the case that these conditions are not simultaneously satisfied, problems occur such as the desired production amount not being obtained, a variation in the number average molecular weight of more than approximately 200 arising, stable production not being possible for 1,000 hours, or discoloration being prone to occur.

The reason why it has become possible to produce the aromatic polycarbonate on an industrial scale with such excellent effects in step (III) is not clear, but this is supposed to be that, in addition to the reasons described above, a composite effect arises when the above conditions are combined. For example, it is supposed that this is because if the guides having the high surface area satisfying the above formulae (16) and (17) are used, then the molten prepolymer

can be polymerized at a relatively low temperature, and a large amount of the high-quality aromatic polycarbonate having a desired molecular weight can be produced; moreover, the tapered bottom casing satisfying the above formula (15) makes it possible to shorten the time taken for the large amount of the produced the high-quality aromatic polycarbonate falling down from the guides to reach the discharge port, whereby the heat history of the produced aromatic polycarbonate can be reduced.

Note that this technology for production on an industrial scale can only be established through prolonged operation using the large-scale production equipment, and it goes without saying that the cost of the production equipment here is an important factor that must be taken into consideration. Another effect of the present invention is that the equipment cost of the industrial production equipment can be reduced by using the guide-contacting downflow type polymerization apparatus satisfying the above formulae (13), (14), (15), (16) and (17).

Ranges required for dimensions, angles and so on for the guide-contacting downflow type polymerization apparatus used in step (III) have been described above, but more preferable ranges are as follows. A more preferable range for the internal sectional area A ($m^2$) taken through the horizontal plane of the side casing in the polymerization reaction zone is $0.8 \leq A \leq 250$, with $1 \leq A \leq 200$ being yet more preferable.

Moreover, a more preferable range for the ratio between A ($m^2$) and the internal sectional area B ($m^2$) taken through the horizontal plane of the aromatic polycarbonate discharge port is $25 \leq A / B \leq 900$, with $30 \leq A / B \leq 800$ being yet more preferable.

Moreover, a more preferable range for the internal angle C (°) of the tapered bottom casing in the polymerization reaction zone to the side casing thereabove is $125 \leq C \leq 160$, with $135 \leq C \leq 165$ being yet more preferable. Note that in the case of progressively increasing the polymerization degree using a plurality of guide-contacting downflow type polymerization apparatuses, taking the angles for the guide-contacting downflow type polymerization apparatuses to be C1, C2, C3 respectively, it is preferable to make $C1 \leq C2 \leq C3 \leq ....$

Moreover, the required length h (cm) of each of the guides varies with factors such as the polymerization degree of the starting material prepolymer, the polymerization temperature and pressure, and the polymerization degree and amount of the aromatic polycarbonate or prepolymer to be produced using the polymerization apparatus, but a more preferable range is $200 \leq h \leq 3000$, with $250 \leq h \leq 2500$ being yet more preferable. It is particularly preferable for h to satisfy the following formula (24);

$$400 < h \leq 2500 \qquad (24).$$

Moreover, the required total external surface area S ($m^2$) of the guides also varies with factors such as the above, but a more preferable range is $4 \leq S \leq 40000$, with $10 \leq S \leq 30000$ being yet more preferable. A particularly preferable range is $15 \leq S \leq 20000$. In the present invention, the "total external surface area of the guide" means the total surface area of the guides with which the molten prepolymer contacts when flowing down; for example, in the case of the guide such as a pipe, this means the surface area of the outside of the pipe, the surface area of the surface inside the pipe over which the molten prepolymer does not flow down not being included.

In the guide-contacting downflow type polymerization apparatus used in step (III), the shape of the inside section taken through the horizontal plane of the side casing in the polymerization reaction zone may be any shape, for example polygonal, elliptical or circular. Operation is generally carried out with the polymerization reaction zone under a reduced pressure, and hence any shape is acceptable so long as this reduced pressure can be withstood, but the circular shape or the shape close thereto is preferable. The side casing in the polymerization reaction zone in the present invention is thus preferably cylindrical. In this case, it is preferable for the tapered conical bottom casing to be connected to the lower portion of the cylindrical side casing, with the cylindrical aromatic polycarbonate discharge port being provided in the lowermost portion of this bottom casing. Taking the cylindrical portion of the side casing to have an inside diameter D (cm) and a length L (cm), and taking the discharge port to have an inside diameter d (cm), D, L and d preferably satisfy the following formulae (20), (21), (22) and (23);

$$100 \leq D \leq 1800 \qquad (20)$$

$$5 \leq D / d \leq 50 \qquad (21)$$

$$0.5 \leq L / D \leq 30 \qquad (22),$$

and

$$h - 20 \leq L \leq h + 300 \qquad (23).$$

For the guide-contacting downflow type polymerization apparatus, a more preferable range for D (cm) is 150 $\leq$ D $\leq$ 1500, with 200 $\leq$ D $\leq$ 1200 being yet more preferable. Moreover, a more preferable range for D / d is 6 $\leq$ D/d $\leq$ 45, with 7 $\leq$ D/d $\leq$ 40 being yet more preferable. Moreover, a more preferable range for L / D is 0.6 $\leq$ L/D $\leq$ 25, with 0.7 $\leq$ L/d $\leq$ 20 being yet more preferable. Moreover, a more preferable range for L (cm) is h - 10 $\leq$ L $\leq$ h + 250, with h $\leq$ L $\leq$ h + 200 being yet more preferable.

The precise reason why the high-quality high-performance aromatic polycarbonate having excellent mechanical properties with no discoloration can be produced stably with no variation in molecular weight for a prolonged period of time on an industrial scale with a fast polymerization rate in step (III) is not clear, but is thought to be as follows. That is, according to the guide-contacting downflow type polymerization process used in step (III), the starting material molten prepolymer is led from the receiving port 1 via the feeding zone 3 and the perforated plate 2 to the guides 4, and then has the polymerization degree thereof increased while flowing down along the guides. Effective internal stirring and surface renewal of the molten prepolymer are carried out as the molten prepolymer flows down along the guides, and thus removal of phenol or the like is carried out effectively, whereby the polymerization proceeds at a fast rate. The melt viscosity increases as the polymerization proceeds, and hence the adhesion force of the flowing down melt material to the guides increases, and thus the amount of the molten matter stuck to each guide increases toward the bottom of the guide. This means that the residence time of the molten prepolymer on the guide, i.e. the polymerization reaction time, increases. Moreover, for the molten prepolymer, which flows down under its own weight while being supported on the guide, the surface area per unit weight is very high, and hence surface renewal is carried out efficiently. Increase of molecular weight in the latter half of polymerization, which has been impossible with prior mechanical stirring type polymerization apparatuses, can thus be attained easily. This is one of the excellent characteristic features of the polymerization apparatus used in step (III).

The only reason that the amount of the molten matter stuck to the guides increases in the latter half of the polymerization below a middle portion of each of the guides is the adhesive holding force, which corresponds to the melt viscosity, and hence approximately the same amount of the molten matter having approximately the same melt viscosity is supported at the same height on each of the guides. Meanwhile, the molten material is continuously fed onto the guides from the top thereof, and hence molten material of increased polymerization degree having approximately the same melt viscosity continuously falls down into the bottom portion of the casing from the lower ends of the guides. That is, aromatic polycarbonate of approximately the same polymerization degree produced while flowing down the guides collects in the tapered bottom portion of the casing, and hence aromatic polycarbonate with no variation in molecular weight can be continuously produced. This is another excellent characteristic feature of the polymerization apparatus used in the present invention. The aromatic polycarbonate collected in the tapered bottom portion of the casing is continuously withdrawn through the discharge port 7 by a discharging pump 8, and is then generally continuously pelletized via an extruder. In this case, additives such as a stabilizer and a weatherproofing agent may be added in the extruder.

The perforated plate in the guide-contacting downflow type polymerization apparatus used in step (III) is generally selected from a flat plates, a corrugated plate, and a plate that is thickened in a middle portion thereof; the shape of the perforated plate is generally selected from shapes such as circular, oval, triangular, and polygonal. The shape of each of the holes in the perforated plate is generally selected from shapes such as circular, oval, triangular, slit-shaped, polygonal, and star-shaped. The sectional area of each of the holes is generally in a range of from 0.01 to 100 $cm^2$, preferably from 0.05 to 10 $cm^2$, particularly preferably from 0.1 to 5 $cm^2$. The spacing between holes, specifically the distance between hole centers, is generally in a range of from 1 to 500 mm, preferably from 25 to 100 mm. The holes in the perforated plate may be holes that penetrate through the perforated plate, or alternatively pipes may be installed in the perforated plate. Moreover, the holes may be tapered.

The "guides" in the guide-contacting downflow type polymerization apparatus used in step (III) means a member for each of which the ratio of the length in a direction perpendicular to a horizontal section relative to the average length of the outer periphery of this horizontal section is very high. This ratio is generally in a range of from 10 to 1,000,000, preferably from 50 to 100,000. The shape of the horizontal section is generally selected from shapes such as circular, oval, triangular, square, polygonal, and star-shaped. The shape of this section may be constant, or may vary, in the

length direction. Moreover, each of the guides may be hollow. Each of the guides may be a single wire, a single thin rod, a single thin pipe made to be such that the molten prepolymer cannot enter therein, or the like, or a plurality of such guides may be combined using a method such as twisting together. Moreover, the guides may form a mesh-like structure, or a punching plate-like structure. The surface of each guide may be smooth, or may be uneven, and may have projections or the like in places. Preferable guides are cylindrical ones such as wires or thin rods, thin pipes as described above, mesh-like guide structures, and punching plate-like guide structures.

The guides may themselves have a heating source such as a heating medium or an electrical heater therein, but guides not having such a heating source therein are particularly preferable since then there is no risk of thermal deterioration of the prepolymer or aromatic polycarbonate on the surface of the guides.

In the guide-contacting downflow type polymerization apparatus used in the present invention, which enables production of the high-quality aromatic polycarbonate on the industrial scale (in terms of production amount, prolonged stable production, etc.), a particularly preferable guide structure is one of a type in which a plurality of guides each having the form of a wire or a thin rod or a thin pipe as described above are joined together at suitable vertical intervals using transverse supports from the top to the bottom of the guides. Examples are metal mesh-like guide structures in which a plurality of guides each having the form of a wire or a thin rod or a thin pipe as described above are fixed together using transverse supports from the top to the bottom of the guides at suitable vertical intervals, for example intervals in a range of from 1 to 200 cm, a three-dimensional guide structure in which a plurality of such mesh-like guide structures are arranged in front of and behind one another and joined together using transverse supports at suitable vertical intervals, for example intervals in a range of from 1 to 200 cm, or a jungle-gym-like three-dimensional guide structure in which a plurality of guides each having the form of a wire or a thin rod or a thin pipe as described above are fixed together in front of and behind, and left and right of, one another using transverse supports at suitable vertical intervals, for example intervals in a range of from 1 to 200 cm. The transverse supports are not only useful for keeping the intervals between the guides approximately constant, but are also useful for increasing the overall strength of flat or curved guides, or guides forming a three-dimensional structure. The supports may be of the same material as the guides, or a different material.

For the guide-contacting downflow type polymerization apparatus, in the case that each guide is cylindrical, or has the form of a pipe made to be such that the molten prepolymer cannot enter therein, with an outside diameter r (cm), r preferably satisfies the following formula (25);

$$0.1 \leq r \leq 1 \qquad (25).$$

The guides promote polymerization of the molten prepolymer as the molten prepolymer flows down the guides, and also have a function of holding the molten prepolymer for a certain period of time. This holding time is related to the polymerization reaction time. As described above, as the polymerization proceeds, the melt viscosity of the molten prepolymer progressively increases, and hence the holding time and the amount held progressively increase. For a given melt viscosity, the amount of the molten prepolymer held by the guides varies with the external surface area of the guides, i.e. in the case of cylindrical or pipe-shaped guides, with the outside diameter.

Moreover, the guides installed in the polymerization apparatus used in the present invention must be strong enough to support their own weight plus the weight of the molten prepolymer being held. For this reason, the thickness of the guides is important, and in the case of cylindrical or pipe-shaped guides, the above formula (25) is preferably satisfied. If r is less than 0.1, then prolonged stable operation becomes difficult in terms of the strength, whereas if r is greater than 1, then the guides themselves become very heavy, and hence there are problems such as having to make the perforated plate very thick so as to hold the guides in the polymerization apparatus, and moreover there is an increase in parts where the amount of the molten prepolymer held is too high, resulting in problems such as variation in the molecular weight increasing. For such reasons, a more preferable range for r is $0.15 \leq r \leq 0.8$, with $0.2 \leq r \leq 0.6$ being yet more preferable.

A preferable material for the guides is one selected from metals such as stainless steel, carbon steel, hastelloy, nickel, titanium, chromium, aluminum, and other alloys, highly heat-resistant polymeric materials, and so on. Stainless steel is particularly preferable. Moreover, surfaces of the guides may be subjected to any of various treatments as necessary such as plating, lining, passivation treatment, acid washing, or washing with phenol.

There are no particular limitations on the positional relationship between the guides and the perforated plate or the positional relationship between the guides and the holes in the perforated plate so long as the molten prepolymer can contact and flow down the guides. The guides and the perforated plate may be in contact with one another, or not in contact with one another. Although there is no such limitation, it is preferable to install the guides in correspondence with the holes in the perforated plate. The reason for this is that design may then be carried out such that the molten prepolymer falling down from the perforated plate contacts the guides in suitable positions. Specific preferable examples

of installing the guides in correspondence with the holes in the perforated plate include: (1) a method in which the upper end of each guide is fixed to an upper inner wall of the polymerization apparatus, and the guides are provided such that each guide penetrates through the vicinity of a nearly central portion of a hole in the perforated plate; (2) a method in which the upper end of each guide is fixed to a peripheral portion at an upper end of a hole in the perforated plate, and the guides are provided such that each guide penetrates through a hole in the perforated plate; and (3) a method in which the upper end of each guide is fixed to the underside of the perforated plate.

Examples of the methods for making the molten prepolymer pass through the perforated plate and flow down along the guides include a method in which the molten prepolymer is allowed to flow down through its own weight or the liquid head, and a method in which the molten prepolymer has pressure applied thereto using a pump or the like and is thus extruded from the perforated plate. A preferable method is one in which a predetermined amount of the starting material molten prepolymer is fed into the feeding zone of the polymerization apparatus under a pressure using a feed pump, and then the molten prepolymer thus led onto the guides via the perforated plate flows down along the guides under its own weight. The molten prepolymer is generally continuously fed into the guide-contacting downflow type polymerization apparatus after having been heated to a predetermined polymerization temperature. It is thus preferable for a jacket or the like to be installed on an outer wall of the guide-contacting downflow type polymerization apparatus, and to carry out heating to a predetermined temperature by passing a heating medium or the like through the jacket, thus heating or maintaining the temperature of the molten prepolymer, the prepolymer feeding zone and the perforated plate, and maintaining the temperature of the polymerization reaction zone, the side casing and the tapered bottom casing.

In step (III), the temperature of the reaction in which the molten prepolymer obtained from the aromatic dihydroxy compound and the diaryl carbonate is subjected to the polymerization in the guide-contacting downflow type polymerization apparatus so as to produce the aromatic polycarbonate is generally in a range of from 80 to 350 °C. However, according to the polymerization apparatus used in the present invention, efficient surface renewal is carried out accompanying internal stirring, and hence the polymerization reaction can be made to proceed at a relatively low temperature. A preferable reaction temperature is thus in a range of from 100 to 290°C, more preferably from 150 to 270°C. With a horizontal biaxial stirring type reactor for ultra-high viscosity polymer, which reactor is a conventional polymerization apparatus, it has generally been necessary to stir for a prolonged period of time at a high temperature of not less than 300 °C under a high vacuum of not more than 133 Pa. Moreover, it has not been possible to avoid yellowing due to leaking in of air, and ingress of foreign matter, through a stirring shaft seal. However, with the polymerization apparatus used in the present invention, because there is no mechanical stirring, there is no such stirring seal, and hence there is very little leaking in of air and so on. Furthermore, another characteristic feature of the present invention is that the polymerization can be carried out adequately at a temperature lower by approximately 20 to 50 °C than in the case of the conventional horizontal biaxial stirring type reactor for ultra-high viscosity polymer. This is another big factor in it being possible to produce a high-quality aromatic polycarbonate with no discoloration or deterioration in properties in the present invention.

Moreover, even if the conventional horizontal biaxial stirring type reactor for ultra-high viscosity polymer is used, producing an aromatic polycarbonate of medium viscosity grade or above is impossible due to the ultra-high viscosity of the aromatic polycarbonate, but with the guide-contacting downflow type polymerization apparatus used in the present invention, even a high viscosity grade aromatic polycarbonate can be produced easily. That is, with the guide-contacting downflow type polymerization apparatus according to the present invention, the aromatic polycarbonates of all grades from a disk grade having a relatively low molecular weight up to a high viscosity grade can be produced. This is also a big characteristic feature of the present invention.

In step (III), as the polymerization reaction proceeds, aromatic monohydroxy compound is produced, but this is removed from the reaction system, whereby the reaction rate can be increased. A method in which an inert gas that does not have an adverse effect on the reaction such as nitrogen, argon, helium, carbon dioxide or a lower hydrocarbon gas is introduced into the polymerization apparatus, and the aromatic monohydroxy compound produced is entrained by this gas and thus removed, a method in which the reaction is carried out under a reduced pressure or the like is thus preferably used. Alternatively, a method in which these two methods are used together can also be preferably used. In such a case, there is no need to introduce a large amount of the inert gas into the polymerization apparatus, but rather just enough to maintain an inert gas atmosphere inside the apparatus is sufficient.

Furthermore, a method in which the inert gas is absorbed into the molten prepolymer in advance before the molten prepolymer is fed into the guide-contacting downflow type polymerization apparatus, and then the molten prepolymer having the inert gas absorbed therein is subjected to the polymerization is also preferable.

A preferable reaction pressure in the polymerization apparatus in step (III) varies depending on the type and molecular weight of the aromatic polycarbonate to be produced, the polymerization temperature, and so on, but in the case, for example, of producing the aromatic polycarbonate from the molten prepolymer produced from bisphenol A and diphenyl carbonate, in the case of a number average molecular weight of less than 5,000, the reaction pressure is preferably in a range of from 400 to 3,000 Pa, and in the case of a number average molecular weight in a range of from

5,000 to 10,000, the reaction pressure is preferably in a range of from 50 to 500 Pa. In the case of a number average molecular weight greater than 10,000, the reaction pressure is preferably not more than 300 Pa, particularly preferably in a range of from 20 to 250 Pa.

When carrying out step (III), an aromatic polycarbonate having a desired polymerization degree can be produced using only one such guide-contacting downflow type polymerization apparatus, but depending on the polymerization degree of the molten prepolymer used as the starting material, the produced amount of the aromatic polycarbonate, and so on, a system in which a plurality of such guide-contacting downflow type polymerization apparatuses are linked together so that the polymerization degree is progressively increased may be preferable. In this case, guides and reaction conditions suitable for the polymerization degree of the prepolymer or aromatic polycarbonate to be produced are preferably adopted for each of the polymerization apparatuses individually. For example, in the case of a system in which the first guide-contacting downflow type polymerization apparatus, the second guide-contacting downflow type polymerization apparatus, the third guide-contacting downflow type polymerization apparatus, the fourth guide-contacting downflow type polymerization apparatus ... are used so that the polymerization degree is progressively increased, taking the total external surface areas of the guides possessed by the polymerization apparatuses to be S1, S2, S3, S4 ... respectively, the system can be made to be such that $S1 \geq S2 \geq S3 \geq S4 \geq$ .... Moreover, the polymerization temperature may be the same in each of the polymerization apparatuses, or alternatively may be progressively increased. Furthermore, the polymerization pressure may be progressively reduced in the polymerization apparatuses.

In this sense, in the case, for example, of progressively increasing the polymerization degree using two polymerization apparatuses, i.e. the first guide-contacting downflow type polymerization apparatus and the second guide-contacting downflow type polymerization apparatus, it is preferable to use guides such that the total external surface area S1 ($m^2$) of the guides in the first polymerization apparatus and the total external surface area S2 ($m^2$) of the guides in the second guide-contacting downflow type polymerization apparatus satisfy the following formula (26);

$$1 \leq S1 / S2 \leq 20 \qquad (26).$$

If S1 / S2 is less than 1, then problems arise such as variation in the molecular weight increasing so that prolonged stable production becomes difficult, and it becoming difficult to obtain a predetermined production amount, whereas if S1 / S2 is greater than 20, then the flow rate of the molten prepolymer flowing down the guides in the second polymerization apparatus becomes high, and as result problems arise such as the residence time of the molten prepolymer becoming short so that it becomes difficult to obtain an aromatic polycarbonate of the required molecular weight. For such reasons, a more preferable range is $1.5 \leq S1 / S2 \leq 15$.

In step (III), not less than 1 ton / hr of the aromatic polycarbonate is produced; but because the aromatic monohydroxy compound by-produced in the polymerization reaction is discharged out of the system, the molten prepolymer must be fed into the polymerization apparatus in an amount greater than 1 ton / hr. The amount of the molten prepolymer fed into the polymerization apparatus thus varies depending on the polymerization degree thereof and the polymerization degree of the aromatic polycarbonate to be produced, but is generally in a range of from 1.01 to 1.5 ton / hr per 1 ton / hr of the aromatic polycarbonate to be produced.

The reaction in which the aromatic polycarbonate is produced from the aromatic dihydroxy compound and the diaryl carbonate in step (III) can be carried out without adding a catalyst, but may be carried out in the presence of a catalyst as required so as to increase the polymerization rate. There are no particular limitations on such a catalyst, which may be any used in the field in question. Examples of the catalyst include alkali metal and alkaline earth metal hydroxides such as lithium hydroxide, sodium hydroxide, potassium hydroxide and calcium hydroxide; alkali metal salts, alkaline earth metal salts and quaternary ammonium salts of boron and aluminum hydrides such as lithium aluminum hydride, sodium borohydride and tetramethylammonium borohydride; alkali metal and alkaline earth metal hydrides such as lithium hydride, sodium hydride and calcium hydride; alkali metal and alkaline earth metal alkoxides such as lithium methoxide, sodium ethoxide and calcium methoxide; alkali metal and alkaline earth metal aryloxides such as lithium phenoxide, sodium phenoxide, magnesium phenoxide, and LiO-Ar-OLi and NaO-Ar-ONa (wherein Ar represents an aryl group); alkali metal and alkaline earth metal organic acid salts such as lithium acetate, calcium acetate and sodium benzoate; zinc compounds such as zinc oxide, zinc acetate and zinc phenoxide; boron compounds such as boron oxide, boric acid, sodium borate, trimethyl borate, tributyl borate, triphenyl borate, and ammonium borates represented by $(R^1R^2R^3R^4)NB(R^1R^2R^3R^4)$ and phosphonium borates represented by $(R^1R^2R^3R^4)PB(R^1R^2R^3R^4)$ (wherein $R^1$, $R^2$, $R^3$ and $R^4$ are defined as above); silicon compounds such as silicon oxide, sodium silicate, tetraalkylsilicon compounds , tetraarylsilicon compounds and diphenyl-ethyl-ethoxysilicon; germanium compounds such as germanium oxide, germanium tetrachloride, germanium ethoxide and germanium phenoxide; tin compounds such as tin oxide, dialkyltin oxides, dialkyltin carboxylates, tin acetate, tin compounds in which alkoxy groups or aryloxy groups are bonded to tin such as ethyltin tributoxide, and organotin compounds; lead compounds, such as lead oxide, lead acetate, lead carbonate, basic

lead carbonate, and lead and organolead alkoxides and aryloxides; onium compounds such as quaternary ammonium salts, quaternary phosphonium salts and quaternary arsonium salts; antimony compounds such as antimony oxide and antimony acetate; manganese compounds such as manganese acetate, manganese carbonate and manganese borate; titanium compounds such as titanium oxide, and titanium alkoxides and titanium aryloxides; and zirconium compounds such as zirconium acetate, zirconium oxide, zirconium alkoxides and aryloxides, and zirconium acetylacetone.

In the case of using such the catalyst, one such catalyst may be used, or a plurality may be used in combination. The amount used of the catalyst is generally selected from a range of from $10^{-10}$ to 1 % by weight, preferably from $10^{-9}$ to $10^{-1}$ % by weight, more preferably from $10^{-8}$ to $10^{-2}$ % by weight, based on the starting material aromatic dihydroxy compound. In the case of the melt transesterification method, the polymerization catalyst used remains in the product aromatic polycarbonate, and often has an adverse effect on the polymer properties. It is thus preferable to reduce the amount used of the catalyst as much as possible. According to the process of the present invention, the polymerization can be carried out efficiently, and hence the amount used of the catalyst can be made low. This is one of the characteristic features of the present invention enabling the high-quality aromatic polycarbonate to be produced.

There are no particular limitations on the material of the guide-contacting downflow type polymerization apparatus and piping used in step (III), but this material is generally selected from metals such as stainless steel, carbon steel, hastelloy, nickel, titanium, chromium, and other alloys, highly heat-resistant polymeric materials, and so on. Moreover, the surface of the material may be subjected to any of various treatments as necessary such as plating, lining, passivation treatment, acid washing, or washing with phenol. Stainless steel, nickel, and a glass lining are particularly preferable.

In step (III), when producing the prepolymer, and during the polymerization in the guide-contacting downflow type polymerization apparatus, a large amount of the aromatic monohydroxy compound by-produced in the reaction is generally continuously withdrawn in a gaseous form, and condensed into a liquid form and thus recovered. In the present invention, an aromatic monohydroxy compound recycling step (IV) of circulating the aromatic monohydroxy compound by-produced in step (III) back into the diaryl carbonate production step (I) must be carried out. In an industrial production process, recovering all of the by-produced aromatic monohydroxy compound or as much as possible thereof with as little loss as possible, and circulating back and thus reusing this aromatic monohydroxy compound is important. The by-produced aromatic monohydroxy compound by-produced in step (III) and recovered in the present invention generally contains some diaryl carbonate but still has high purity, and hence can be circulated back into the diaryl carbonate production step (I) and thus reused as is. In the case that a small amount of aromatic dihydroxy compound or a trace amount of oligomer is contained in the recovered aromatic monohydroxy compound, it is preferable to remove this high boiling point material by further carrying out distillation before circulating the aromatic monohydroxy compound back into the diaryl carbonate production step (I) for reuse.

The aromatic polycarbonate produced by implementing the system of the present invention has a repeat unit represented by following formula:

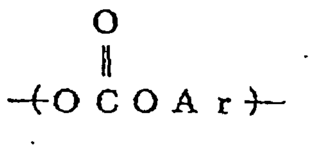

wherein Ar is defined as before.

A particularly preferable aromatic polycarbonate is one containing not less than 85 mol% of the repeat unit represented by following formula out of all the repeat units:

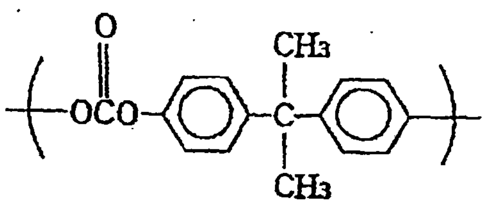

Moreover, a terminal group of the aromatic polycarbonate produced by implementing the process of the present invention is generally a hydroxy group or an aryl carbonate group represented by following formula;

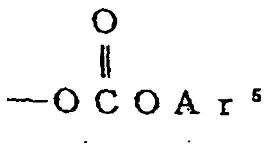

wherein $Ar^5$ is the same as $Ar^3$ or $Ar^4$ described above.

There are no particular limitations on the ratio of hydroxy groups to aryl carbonate groups, but this ratio is generally in a range of from 95:5 to 5:95, preferably from 90:10 to 10:90, more preferably from 80:20 to 20:80. An aromatic polycarbonate in which the proportion of phenyl carbonate groups among the terminal groups is not less than 60 mol% is particularly preferable.

The aromatic polycarbonate produced by implementing the process of the present invention may have a main chain thereof partially branched through foreign linkages such as ester linkages or ether linkages. The amount of such foreign linkages is generally in a range of from 0.005 to 2 mol%, preferably from 0.01 to 1 mol%, more preferably from 0.05 to 0.5 mol %, based on the carbonate linkages. Such an amount of foreign linkages is suitable for precision molding since the flow characteristics during melt molding can be improved without other polymer properties being worsened; molding can be carried out even at a relatively low temperature, and a molded article having excellent performance can be produced. The molding cycle can also be shortened, contributing to energy saving during the molding.

The aromatic polycarbonate produced by implementing the process of the present invention hardly contains any impurities, it being possible to produce an aromatic polycarbonate containing 0.001 to 1 ppm of alkali metal and / or alkaline earth metal compounds in terms of the metallic elements therein. This content is preferably in a range of from 0.005 to 0.5 ppm, more preferably from 0.01 to 0.1 ppm. In the case that the content of such metallic elements is not more than 1 ppm, preferably not more than 0.5 ppm, more preferably 0.1 ppm, there are no adverse effects on the properties of the product aromatic polycarbonate, and hence the aromatic polycarbonate produced through the present invention is of high quality.

The aromatic polycarbonate produced by implementing the process of the present invention is particularly preferably one produced using the aromatic dihydroxy compound and the diaryl carbonate each not containing a halogen, the halogen content generally being not more than 10 ppb. According to the process of the present invention, even an aromatic polycarbonate having a halogen content of not more than 5 ppb, more preferably not more than 1 ppb, can be produced, and hence a product having very high quality can be obtained.

It is clear from the Examples, described below, that in the process of the present invention, it is because the specified polymerization apparatus is used that the aromatic polycarbonate can be produced stably for a prolonged period of time with no variation in molecular weight.

Examples

Following is a more detailed description of the present invention through Examples. However, the present invention is not limited to the following Examples.

Number average molecular weight (Mn): Measurement was carried out by a gel permeation chromatography (GPC) method using tetrahydrofuran as a transporting solvent, and the number average molecular weight (Mn) was determined using a converted molecular weight calibration curve given by the following formula obtained using standard monodisperse polystyrene;

$$M_{PC} = 0.3591\ M_{PS}^{1.0388}$$

wherein $M_{PC}$ represents the molecular weight of the aromatic polycarbonate, and $M_{PS}$ represents the molecular weight of the polystyrene.

Color: Using an injection molding machine, a length 50 mm × width 50 mm × thickness 3.2 mm test piece was continuously molded from the aromatic polycarbonate at a cylinder temperature of 290 °C and a mold temperature of 90 °C. The color tone of the test piece obtained was measured in accordance with the CIELAB method (Commission Internationale de l'Eclairage 1976 L*a*b* Diagram), the yellowness being given by the b* value.

Tensile elongation: Using an injection molding machine, the aromatic polycarbonate was injection-molded at a cylinder temperature of 290 °C and a mold temperature of 90 °C. The tensile elongation (%) of the 3.2 mm-thick test piece obtained was measured in accordance with ASTM D638.

An amount of foreign linkages was measured using the method described in WO97/32916, the alkali metal / alkaline earth metal content was measured using an ICP method, and the halogen content was measured using an ion

chromatography method.

Example 1:

(1) Step (I) of continuously producing diphenyl carbonate

<First continuous multi-stage distillation column 101>

A continuous multi-stage distillation column as shown in FIG. 1 having $L_1$ = 3300 cm, $D_1$ = 500 cm, $L_1$ / $D_1$ = 6.6, $n_1$ = 80, $D_1$ / $d_{11}$ = 17, and $D_1$ / $d_{12}$ = 9 was used. In this Example, sieve trays each having a cross-sectional area per hole of approximately 1.5 $cm^2$ and a number of holes of approximately 250 / $m^2$ were used as the internals.

<Second continuous multi-stage distillation column 201 >

A continuous multi-stage distillation column as shown in FIG. 2 having $L_2$ = 3100 cm, $D_2$ = 500 cm, $L_2$ / $D_2$ = 6.2, $n_2$ = 30, $D_2$ / $d_{21}$ = 3.85, and $D_2$ / $d_{22}$ = 11.1 was used. In this Example, as the internals, two sets of Mellapak (11 theoretical stages in total) were installed in the upper portion, and sieve trays each having a cross-sectional area per hole of approximately 1.3 $cm^2$ and a number of holes of approximately 250 / $m^2$ were used in the lower portion.

<Reactive distillation>

Diphenyl carbonate was produced by carrying out reactive distillation using an apparatus in which the first continuous multi-stage distillation column 101 and the second continuous multi-stage distillation column 201 were connected together as shown in FIG. 3.

A starting material 1 containing phenol and dimethyl carbonate in a weight ratio of phenol / dimethyl carbonate = 1.9 was introduced continuously in a liquid form at a flow rate of 50 ton / hr from an upper inlet 11 of the first continuous multi-stage distillation column 101. On the other hand, a starting material 2 containing dimethyl carbonate and phenol in a weight ratio of dimethyl carbonate / phenol = 3.6 was introduced continuously in a gaseous form at a flow rate of 50 ton / hr from a lower inlet 12 of the first continuous multi-stage distillation column 101. The molar ratio for the starting materials introduced into the first continuous multi-stage distillation column 101 was dimethyl carbonate / phenol = 1.35. The starting materials substantially did not contain halogens (outside the detection limit for the ion chromatography, i.e. not more than 1 ppb). $Pb(OPh)_2$ as a catalyst was introduced from the upper inlet 11 of the first continuous multi-stage distillation column 101 such that a concentration thereof in the reaction liquid would be approximately 100 ppm. Reactive distillation was carried out continuously under the conditions of a temperature at the bottom of the first continuous multi-stage distillation column 101 of 225 °C, a pressure at the top of the column of $7\times10^5$ Pa and a reflux ratio of 0. A first column low boiling point reaction mixture containing methanol, dimethyl carbonate, phenol and so on was continuously withdrawn in a gaseous form from the top 13 of the first column, was passed through a heat exchanger 14, and was withdrawn at a flow rate of 34 ton / hr from an outlet 16. On the other hand, a first column high boiling point reaction mixture containing methyl phenyl carbonate, dimethyl carbonate, phenol, diphenyl carbonate, the catalyst and so on was continuously withdrawn in a liquid form from the bottom 17 of the first column.

A stable steady state was attained after 24 hours. The first column high boiling point reaction mixture was thus then fed continuously as is into the second continuous multi-stage distillation column 201 at a flow rate of 66 ton / hr from a starting material inlet 21 installed between the Mellapak and the sieve trays. The liquid fed into the second continuous multi-stage distillation column 201 contained 18.2 % by weight of methyl phenyl carbonate and 0.8 % by weight of diphenyl carbonate. Reactive distillation was carried out continuously in the second continuous multi-stage distillation column 201 under the conditions of a temperature at the bottom of the column of 210°C, a pressure at the top of the column of $3\times10^4$ Pa, and a reflux ratio of 0.3. It was possible to attain stable steady state operation after 24 hours. A second column low boiling point reaction mixture containing 35 % by weight of dimethyl carbonate and 56 % by weight of phenol was continuously withdrawn from the top 23 of the second column, the flow rate at an outlet 26 being 55.6 ton / hr, and a second column high boiling point reaction mixture containing 38.4 % by weight of methyl phenyl carbonate and 55.6 % by weight of diphenyl carbonate was continuously withdrawn from the bottom 27 of the second column. The second column low boiling point reaction mixture was continuously fed into the first continuous multi-stage distillation column 101 from the inlet 11. At this time, the amounts of fresh dimethyl carbonate and phenol newly fed into the first continuous multi-stage distillation column 101 were adjusted so as to maintain the above compositions and amounts of the starting material 1 and the starting material 2, taking into consideration the composition and amount of the second column low boiling point reaction mixture. It was found that the amount of diphenyl carbonate produced per hour was 5.74 ton. The selectivity for the diphenyl carbonate based on the phenol reacted was 98%.

Prolonged continuous operation was carried out under these conditions. The amounts of diphenyl carbonate

produced per hour after 500 hours, 2000 hours, 4000 hours, 5000 hours, and 6000 hours (excluding the diphenyl carbonate contained in the starting material) were 5.74 ton, 5.75 ton, 5.74 ton, 5.74 ton, and 5.75 ton respectively, and the selectivities were 98%, 98%, 98%, 98%, and 98% respectively, and hence the operation was very stable. Moreover, the aromatic carbonates produced substantially did not contain halogens (not more than 1 ppb).

(2) Step (II) of obtaining high-purity diphenyl carbonate

The second column high boiling point reaction mixture withdrawn from the bottom of the second continuous multi-stage distillation column was continuously fed into a high boiling point matter separating column (length 1700 cm, inside diameter 340 cm, 30 stages), and distillation was carried out continuously with a temperature at the bottom of the column of 206 °C, a pressure at the top of the column of 3800 Pa, and a reflux ratio of 0.6. A column top component continuously withdrawn from the top of the high boiling point matter separating column was continuously fed as is into an inlet of a diaryl carbonate purifying column having an side cut outlet (length 2200 cm, inside diameter 280 cm, 12 stages above inlet, 18 stages between inlet and side cut outlet installed therebelow, 5 stages below side cut outlet). In the diaryl carbonate purifying column, distillation was carried out continuously with a temperature at the bottom of the column of 213 °C, a pressure at the top of the column of 5000 Pa, and a reflux ratio of 1.5. The purity of diphenyl carbonate continuously withdrawn from the side cut outlet was not less than 99.999%, and the halogen content was not more than 1 ppb.

The high-purity diphenyl carbonate obtained in this way was temporarily stored in a molten state in a storage tank.

(3) Step (III) of producing high-quality polycarbonate

Aromatic polycarbonate production was carried out using a guide-contacting downflow type polymerization apparatus as shown in FIG. 5. The material of the polymerization apparatus was all stainless steel. The polymerization apparatus had a cylindrical casing and a tapered conical bottom portion, and was such that L = 1,000 cm, h = 900 cm, D = 500 cm, d = 40 cm, C = 155°, and S = 250 $m^2$. Molten polymer fed in from a feeding port 1 was distributed uniformly by a perforated plate 2 to guides 4. An inert gas feeding port 9 was provided in a lower portion of the polymerization apparatus, and a vacuum vent 6 was provided in an upper portion. There was a jacket on the outside of the polymerization apparatus, heating being carried out using a heating medium.

An aromatic polycarbonate molten prepolymer (number average molecular weight Mn = 4,000) produced from bisphenol A and the high-purity diphenyl carbonate produced in the steps (I) and (II) (molar ratio relative to bisphenol A = 1.05) and held at 260°C was continuously fed into a feeding zone 3 from the feeding port 1 by a feed pump. The molten prepolymer, which was continuously fed into a polymerization reaction zone 5 via the perforated plate 2 in the polymerization apparatus, was subjected to polymerization while flowing down along the guides 4. The polymerization reaction zone 5 was held at 80 Pa via the vacuum vent 6. Produced aromatic polycarbonate entering the tapered bottom portion 11 of the polymerization apparatus from the lower ends of the guides 4 was continuously withdrawn at a flow rate of 5.5 ton / hr from a discharge port 7 by a discharging pump 8 such that the amount of the aromatic polycarbonate residing in the bottom portion was nearly constant.

The number average molecular weight Mn of the aromatic polycarbonate withdrawn from an outlet 12 after 50 hours from commencement of operation was 10,500, and the aromatic polycarbonate had a good color tone (b* = 3.2). Moreover, the tensile elongation was 98%. The values of Mn for the aromatic polycarbonate withdrawn from the outlet 12 after 60 hours, 100 hours, 500 hours, 1,000 hours, 2,000 hours, 3,000 hours, 4,000 hours, and 5,000 hours from commencement of operation were 10,500, 10,550, 10,500, 10,550, 10,500, 10,500, 10,550, and 10,500 respectively, and hence operation was stable.

The aromatic polycarbonate produced as the above had a content of alkali metal and / or alkaline earth metal compounds of 0.04 to 0.05 ppm in terms of the metallic elements therein, and a chlorine content of not more than 1 ppb (outside of the detection limit). Moreover, the content of foreign linkages was 0.12 to 0.15 mol%.

(4) Step (IV) of recycling aromatic monohydroxy compound

A phenol solution containing approximately 10% of diphenyl carbonate and a trace amount of bisphenol A by-produced in step (III) and recovered in a liquid form was continuously fed into a phenol purifying column (length 1500 cm, inside diameter 270 cm, 9 stages). Distillation was carried out continuously with a temperature at the bottom of the column of 185°C, a pressure at the top of the column of 2000 Pa, and a reflux ratio of 0.9. Phenol recovered from the top of the column was temporarily stored in a tank, and then recycled back into step (I). Diphenyl carbonate recovered from a side cut was fed into the high boiling point matter separating column of step (II), and thus recovered in the form of high-purity diphenyl carbonate.

Example 2:

(1) Step (I) of continuously producing diphenyl carbonate

Reactive distillation was carried out under the following conditions using the same apparatus as in Example 1.

A starting material 1 containing phenol and dimethyl carbonate in a weight ratio of phenol / dimethyl carbonate = 1.1 was introduced continuously in a liquid form at a flow rate of 40 ton/hr from the upper inlet 11 of the first continuous multi-stage distillation column 101. On the other hand, a starting material 2 containing dimethyl carbonate and phenol in a weight ratio of dimethyl carbonate / phenol = 3.9 was introduced continuously in a gaseous form at a flow rate of 43 ton / hr from the lower inlet 12 of the first continuous multi-stage distillation column 101. The molar ratio for the starting materials introduced into the first continuous multi-stage distillation column 101 was dimethyl carbonate / phenol = 1.87. The starting materials substantially did not contain halogens (outside the detection limit for the ion chromatography, i.e. not more than 1 ppb). $Pb(OPh)_2$ as a catalyst was introduced from the upper inlet 11 of the first continuous multi-stage distillation column 101 such that a concentration thereof in the reaction liquid would be approximately 250 ppm. Reactive distillation was carried out continuously under conditions of a temperature at the bottom of the first continuous multi-stage distillation column 101 of 235°C, a pressure at the top of the column of $9\times10^5$ Pa and a reflux ratio of 0. A first column low boiling point reaction mixture containing methanol, dimethyl carbonate, phenol and so on was continuously withdrawn in a gaseous form from the top 13 of the first column, was passed through the heat exchanger 14, and was withdrawn at a flow rate of 43 ton / hr from the outlet 16. On the other hand, a first column high boiling point reaction mixture containing methyl phenyl carbonate, dimethyl carbonate, phenol, diphenyl carbonate, the catalyst and so on was continuously withdrawn in a liquid form from the bottom 17 of the first column.

A stable steady state was attained after 24 hours. The first column high boiling point reaction mixture was thus then fed continuously as is into the second continuous multi-stage distillation column 201 at a flow rate of 40 ton / hr from the starting material inlet 21 installed between the Mellapak and the sieve trays. The liquid fed into the second continuous multi-stage distillation column 201 contained 20.7 % by weight of methyl phenyl carbonate and 1.0 % by weight of diphenyl carbonate. Reactive distillation was carried out continuously in the second continuous multi-stage distillation column 201 under conditions of a temperature at the bottom of the column of 205°C, a pressure at the top of the column of $2\times10^4$ Pa, and a reflux ratio of 0.5. It was possible to attain stable steady state operation after 24 hours. A second column low boiling point reaction mixture was continuously withdrawn from the top 23 of the second column, and a second column high boiling point reaction mixture containing 36.2 % by weight of methyl phenyl carbonate and 60.8 % by weight of diphenyl carbonate was continuously withdrawn from the bottom 27 of the second column. The second column low boiling point reaction mixture was continuously fed into the first continuous multi-stage distillation column 101 from the inlet 11. At this time, the amounts of fresh dimethyl carbonate and phenol newly fed into the first continuous multi-stage distillation column 101 were adjusted so as to maintain the above compositions and amounts of the starting material 1 and the starting material 2, taking into consideration the composition and amount of the second column low boiling point reaction mixture. It was found that the amount of diphenyl carbonate produced per hour was 4.03 ton. The selectivity for the diphenyl carbonate based on the phenol reacted was 97%.

Prolonged continuous operation was carried out under these conditions. The amounts of diphenyl carbonate produced per hour after 500 hours, 1000 hours, and 2000 hours were 4.03 ton, 4.03 ton, and 4.04 ton respectively, and the selectivities based on the reacted phenol were 97%, 97%, and 97% respectively, and hence the operation was very stable. Moreover, the aromatic carbonates produced substantially did not contain halogens (not more than 1 ppb).

(2) Step (II) of obtaining high-purity diphenyl carbonate

This was carried out using the same process as in Example 1.

(3) Step (III) of producing high-quality polycarbonate

Using the same polymerization apparatus as in Example 1, an aromatic polycarbonate molten prepolymer (number average molecular weight Mn = 3,500) produced from bisphenol A and the high-purity diphenyl carbonate produced in the steps (I) and (II) (molar ratio relative to bisphenol A = 1.05) was continuously fed into the feeding zone 3 from the feeding port 1 by the feed pump. Polymerization was carried out to produce an aromatic polycarbonate using the same process as in Example 1, except that the pressure in the polymerization reaction zone was held at 100 Pa. The values of Mn for the aromatic polycarbonate discharged from a discharge port 12 after 50 hours, 100 hours, 500 hours, 1,000 hours, 2,000 hours, 3,000 hours, 4,000 hours, and 5,000 hours from commencement of operation were 7,600, 7,600, 7,650, 7,600, 7,650, 7,650, 7,600, and 7,600 respectively, and hence operation was stable.

The aromatic polycarbonate produced as the above had a content of alkali metal and / or alkaline earth metal compounds of 0.03 to 0.04 ppm in terms of the metallic elements therein, and a chlorine content of not more than 1 ppb

(outside of the detection limit). Moreover, the content of foreign linkages was 0.08 to 0.1 mol%.

(4) Step (IV) of recycling aromatic monohydroxy compound

This was carried out using the same process as in Example 1.

Example 3:

(1) Step (I) of continuously producing diphenyl carbonate

Reactive distillation was carried out under the following conditions using the same apparatus as in Example 1 except that the cross-sectional area per hole of each of the sieve trays in the second continuous multi-stage distillation column 201 was made to be approximately 1.8 $cm^2$.

A starting material 1 containing phenol and dimethyl carbonate in a weight ratio of phenol / dimethyl carbonate = 1.7 was introduced continuously in a liquid form at a flow rate of 86 ton / hr from the upper inlet 11 of the first continuous multi-stage distillation column 101. On the other hand, a starting material 2 containing dimethyl carbonate and phenol in a weight ratio of dimethyl carbonate / phenol = 3.5 was introduced continuously in a gaseous form at a flow rate of 90 ton / hr from the lower inlet 12 of the first continuous multi-stage distillation column 101. The molar ratio for the starting materials introduced into the first continuous multi-stage distillation column 101 was dimethyl carbonate / phenol = 1.44. The starting materials substantially did not contain halogens (outside the detection limit for the ion chromatography, i.e. not more than 1 ppb). $Pb(OPh)_2$ as a catalyst was introduced from the upper inlet 11 of the first continuous multi-stage distillation column 101 such that a concentration thereof in the reaction liquid would be approximately 150 ppm. Reactive distillation was carried out continuously under conditions of a temperature at the bottom of the first continuous multi-stage distillation column 101 of 220°C, a pressure at the top of the column of $8\times10^5$ Pa and a reflux ratio of 0. A first column low boiling point reaction mixture containing methanol, dimethyl carbonate, phenol and so on was continuously withdrawn in a gaseous form from the top 13 of the first column, was passed through the heat exchanger 14, and was withdrawn at a flow rate of 82 ton / hr from the outlet 16. On the other hand, a first column high boiling point reaction mixture containing methyl phenyl carbonate, dimethyl carbonate, phenol, diphenyl carbonate, the catalyst and so on was continuously withdrawn in a liquid form from the bottom 17 of the first column.

A stable steady state was attained after 24 hours. The first column high boiling point reaction mixture was thus then fed continuously as is into the second continuous multi-stage distillation column 201 at a flow rate of 94 ton / hr from the starting material inlet 21 installed between the Mellapak and the sieve trays. The liquid fed into the second continuous multi-stage distillation column 201 contained 16.0 % by weight of methyl phenyl carbonate and 0.5 % by weight of diphenyl carbonate. Reactive distillation was carried out continuously in the second continuous multi-stage distillation column 201 under the conditions of a temperature at the bottom of the column of 215°C, a pressure at the top of the column of $2.5\times10^4$ Pa, and a reflux ratio of 0.4. It was possible to attain stable steady state operation after 24 hours. A second column low boiling point reaction mixture was continuously withdrawn from the top 23 of the second column, and a second column high boiling point reaction mixture containing 35.5 % by weight of methyl phenyl carbonate and 59.5 % by weight of diphenyl carbonate was continuously withdrawn from the bottom 27 of the second column. The second column low boiling point reaction mixture was continuously fed into the first continuous multi-stage distillation column 101 from the inlet 11. At this time, the amounts of fresh dimethyl carbonate and phenol newly fed into the first continuous multi-stage distillation column 101 were adjusted so as to maintain the above compositions and amounts of the starting material 1 and the starting material 2, taking into consideration the composition and amount of the second column low boiling point reaction mixture. It was found that the amount of diphenyl carbonate produced per hour was 7.28 ton. The selectivity for the diphenyl carbonate based on the reacted phenol was 98%.

Prolonged continuous operation was carried out under these conditions. The amounts of diphenyl carbonate produced per hour after 500 hours, 1000 hours, and 2000 hours were 7.28 ton, 7.29 ton, and 7.29 ton respectively, and the selectivities based on the reacted phenol were 98%, 98%, and 98% respectively, and hence the operation was very stable. Moreover, the aromatic carbonates produced substantially did not contain halogens (not more than 1 ppb).

(2) Step (II) of obtaining high-purity diphenyl carbonate

This was carried out using the same process as in Example 1.

(3) Step (III) of producing high-quality polycarbonate

Aromatic polycarbonate production was carried out using a polymerization apparatus in which two guide-contacting downflow type polymerization apparatuses as shown in FIG. 5 were arranged in series. The material of each

of the polymerization apparatuses was all stainless steel. The first guide-contacting downflow type polymerization apparatus had a cylindrical casing and a tapered conical bottom portion, and was such that L = 950 cm, h = 850 cm, D = 400 cm, d = 20 cm, C = 150°, and S = 750 $m^2$. The second polymerization apparatus was the same as the polymerization apparatus used in Example 1.

An aromatic polycarbonate molten prepolymer (number average molecular weight Mn = 2,500) produced from bisphenol A and the high-purity diphenyl carbonate produced in the steps (I) and (II) (molar ratio relative to bisphenol A = 1.06) was continuously fed into the feeding zone 3 from the feeding port 1 of the first polymerization apparatus by the feed pump. The molten prepolymer, which was continuously fed into the polymerization reaction zone via the perforated plate 2 in the first polymerization apparatus, was subjected to polymerization while flowing down along the guides 4. The polymerization reaction zone of the first polymerization apparatus was held at a pressure of 800 Pa via the vacuum vent 6. Aromatic polycarbonate molten prepolymer of increased polymerization degree (number average molecular weight Mn = 5,500) entering the tapered bottom portion 11 of the polymerization apparatus from the bottom end of the guides 4 was continuously withdrawn at a constant flow rate from the discharge port 7 by the discharging pump 8 such that the amount of the aromatic polycarbonate molten prepolymer residing in the bottom portion was nearly constant. This molten prepolymer was continuously fed into the feeding zone 3 from the feeding port 1 of the second polymerization apparatus by a feed pump. The molten prepolymer, which was continuously fed into the polymerization reaction zone via the perforated plate 2 in the second polymerization apparatus, was subjected to polymerization while flowing down along the guides 4. The polymerization reaction zone of the second polymerization apparatus was held at a pressure of 50 Pa via the vacuum vent 6. Produced aromatic polycarbonate entering the tapered bottom portion 11 of the second polymerization apparatus from the lower ends of the guides 4 was continuously withdrawn at a flow rate of 6 ton / hr from the discharge port 7 by the discharging pump 8 such that the amount of the aromatic polycarbonate residing in the bottom portion was nearly constant.

The number average molecular weight Mn of the aromatic polycarbonate withdrawn from the outlet 12 of the second polymerization apparatus after 50 hours from commencement of operation was 11,500, and the aromatic polycarbonate had a good color tone (b* = 3.2). Moreover, the tensile elongation was 99%. The values of Mn for the aromatic polycarbonate withdrawn from the outlet 12 after 60 hours, 100 hours, 500 hours, 1,000 hours, 2,000 hours, 3,000 hours, 4,000 hours, and 5,000 hours from commencement of operation were 11,500, 11,550, 11,500, 11,550, 11,500, 11,500, 11,550, and 11,500 respectively, and hence operation was stable.

The aromatic polycarbonate produced as the above had a content of alkali metal and / or alkaline earth metal compounds of 0.03 to 0.05 ppm in terms of the metallic elements therein, and a chlorine content of not more than 1 ppb (outside of the detection limit). Moreover, the content of foreign linkages was 0.11 to 0.16 mol%.

(4) Step (IV) of recycling aromatic monohydroxy compound

This was carried out using the same process as in Example 1.

Industrial Applicability

According to the present invention, there is provide a specific process that enables a high-quality high-performance aromatic polycarbonate having excellent mechanical properties and no discoloration to be produced industrially in a large amount (e.g. not less than 1 ton / hr) stably for a prolonged period of time (e.g. not less than 1000 hours, preferably not less than 3000 hours, more preferably not less than 5000 hours) from a dialkyl carbonate and an aromatic dihydroxy compound.

## Claims

**1.** An industrial process for the production of a high-quality aromatic polycarbonate in which an aromatic polycarbonate is continuously produced from a dialkyl carbonate and an aromatic dihydroxy compound, the process comprising the steps of:

(I) continuously producing a diaryl carbonate by taking the dialkyl carbonate and the aromatic monohydroxy compound as a starting material, continuously feeding the starting material into a first continuous multi-stage distillation column in which a catalyst is present, carrying out reaction and distillation simultaneously in said first column, continuously withdrawing a first column low boiling point reaction mixture containing a produced alcohol from an upper portion of said first column in a gaseous form, continuously withdrawing a first column high boiling point reaction mixture containing a produced alkyl aryl carbonate from a lower portion of said first column in a liquid form, continuously feeding said first column high boiling point reaction mixture into a second continuous

multi-stage distillation column in which a catalyst is present, carrying out reaction and distillation simultaneously in said second column, continuously withdrawing a second column low boiling point reaction mixture containing a produced dialkyl carbonate from an upper portion of said second column in a gaseous form, continuously withdrawing a second column high boiling point reaction mixture containing a produced diaryl carbonate from a lower portion of said second column in a liquid form, and continuously feeding the second column low boiling point reaction mixture containing the dialkyl carbonate into the first continuous multi-stage distillation column;
(II) purifying said diaryl carbonate so as to obtain a high-purity diaryl carbonate;
(III) reacting said aromatic dihydroxy compound and said high-purity diaryl carbonate together so as to produce an aromatic polycarbonate molten prepolymer, and producing an aromatic polycarbonate using a guide-contacting downflow type polymerization apparatus in which said molten prepolymer is made to flow down along surfaces of guides, and said molten prepolymer is polymerized while flowing down; and
(IV) circulating the aromatic monohydroxy compound by-produced in step (III) back into the diaryl carbonate production step (I) so as to recycle the aromatic monohydroxy compound;

wherein:

(a) said first continuous multi-stage distillation column comprises a structure having a cylindrical trunk portion having a length $L_1$ (cm) and an inside diameter $D_1$ (cm), and having an internal with number of stages $n_1$ thereinside, and has a gas outlet having an inside diameter $d_{11}$ (cm) at a top of the column or in an upper portion of the column near to the top, a liquid outlet having an inside diameter $d_{12}$ (cm) at a bottom of the column or in a lower portion of the column near to the bottom, at least one first inlet provided in the upper portion and / or a middle portion of the column below said gas outlet, and at least one second inlet provided in the middle portion and / or the lower portion of the column above said liquid outlet, wherein $L_1$, $D_1$, $L_1$ / $D_1$, $n_1$, $D_1$ / $d_{11}$, and $D_1$ / $d_{12}$ respectively satisfy the following formulae (1) to (6):

$$1500 \leq L_1 \leq 8000 \qquad (1),$$

$$100 \leq D_1 \leq 2000 \qquad (2),$$

$$2 \leq L_1 / D_1 \leq 40 \qquad (3),$$

$$20 \leq n_1 \leq 120 \qquad (4),$$

$$5 \leq D_1 / d_{11} \leq 30 \qquad (5),$$

$$3 \leq D_1 / d_{12} \leq 20 \qquad (6);$$

and
(b) said second continuous multi-stage distillation column comprises a structure having a cylindrical trunk portion having a length $L_2$ (cm) and an inside diameter $D_2$ (cm), and having an internal with number of stages $n_2$ thereinside, and has a gas outlet having an inside diameter $d_{21}$ (cm) at a top of the column or in an upper portion of the column near to the top, a liquid outlet having an inside diameter $d_{22}$ (cm) at a bottom of the column or in an lower portion of the column near to the bottom, at least one third inlet provided in the upper portion and / or a middle portion of the column below said gas outlet, and at least one fourth inlet provided in the middle portion and / or the lower portion of the column above the liquid outlet, wherein $L_2$, $D_2$, $L_2$ / $D_2$, $n_2$, $D_2$ / $d_{21}$, and $D_2$ /

$d_{22}$ respectively satisfy the following formulae (7) to (12):

$$1500 \leq L_2 \leq 8000 \quad (7),$$

$$100 \leq D_2 \leq 2000 \quad (8),$$

$$2 \leq L_2 / D_2 \leq 40 \quad (9),$$

$$10 \leq n_2 \leq 80 \quad (10),$$

$$2 \leq D_2 / d_{21} \leq 15 \quad (11),$$

$$5 \leq D_2 / d_{22} \leq 30 \quad (12);$$

and
(c) said guide-contacting downflow type polymerization apparatus comprises:

(1) an apparatus having a molten prepolymer receiving port, a perforated plate, a polymerization reaction zone having a plurality of guides that extend downward from said perforated plate provided in a space surrounded by said perforated plate, a side casing and a tapered bottom casing, a molten prepolymer feeding zone for feeding a molten prepolymer via said perforated plate onto the guides in the polymerization reaction zone, a vacuum vent provided in said polymerization reaction zone, an aromatic polycarbonate discharge port provided in a lowermost portion of the tapered bottom casing, and an aromatic polycarbonate discharge pump connected to said discharge port,
wherein:
(2) an internal sectional area A ($m^2$) taken through a horizontal plane of the side casing in said polymerization reaction zone satisfies the following formula (13),

$$0.7 \leq A \leq 300 \quad (13),$$

(3) a ratio between said A ($m^2$) and an internal sectional area B ($m^2$) taken through a horizontal plane of the aromatic polycarbonate discharge port satisfies the following formula (14),

$$20 \leq A / B \leq 1000 \quad (14),$$

(4) the tapered bottom casing in said polymerization reaction zone is connected at an internal angle C (°) to the side casing thereabove, wherein said angle C (°) satisfies the following formula (15),

$$120 \leq C \leq 165 \quad (15),$$

(5) a length h (cm) of said guides satisfies the following formula (16),

$$150 \leq h \leq 5000 \qquad (16),$$

and
(6) a total external surface area S ($m^2$) of said guides satisfies the following formula (17),

$$2 \leq S \leq 50000 \qquad (17).$$

**2.** The process according to Claim 1, wherein not less than 1 ton / hr of the aromatic polycarbonate is produced.

**3.** The process according to Claim 1 or 2, wherein said $d_{11}$ and said $d_{12}$ satisfy the following formula (18), and said $d_{21}$ and said $d_{22}$ satisfy the following formula (19),

$$1 \leq d_{12} / d_{11} \leq 5 \qquad (18)$$

$$1 \leq d_{21} / d_{22} \leq 6 \qquad (19).$$

**4.** The process according to any one of Claims 1 to 3, wherein $L_1$, $D_1$, $L_1 / D_1$, $n_1$, $D_1 / d_{11}$, and $D_1 / d_{12}$ for said first continuous multi-stage distillation column satisfy respectively $2000 \leq L_1 \leq 6000$, $150 \leq D_1 \leq 1000$, $3 \leq L_1 / D_1 \leq 30$, $30 \leq n_1 \leq 100$, $8 \leq D_1 / d_{11} \leq 25$, and $5 \leq D_1 / d_{12} \leq 18$, and $L_2$, $D_2$, $L_2 / D_2$, $n_2$, $D_2 / d_{21}$, and $D_2 / d_{22}$ for said second continuous multi-stage distillation column satisfy respectively $2000 \leq L_2 \leq 6000$, $150 \leq D_2 \leq 1000$, $3 \leq L_2 / D_2 \leq 30$, $15 \leq n_2 \leq 60$, $2.5 \leq D_2 / d_{21} \leq 12$, and $7 \leq D_2 / d_{22} \leq 25$.

**5.** The process according to any one of Claims 1 to 4, wherein $L_1$, $D_1$, $L_1 / D_1$, $n_1$, $D_1 / d_{11}$, and $D_1 / d_{12}$ for said first continuous multi-stage distillation column satisfy respectively $2500 \leq L_1 \leq 5000$, $200 \leq D_1 \leq 800$, $5 \leq L_1 / D_1 \leq 15$, $40 \leq n_1 \leq 90$, $10 \leq D_1 / d_{11} \leq 25$, and $7 \leq D_1 / d_{12} \leq 15$, and $L_2$, $D_2$, $L_2 / D_2$, $n_2$, $D_2 / d_{21}$, and $D_2 / d_{22}$ for said second continuous multi-stage distillation column satisfy respectively $2500 \leq L_2 \leq 5000$, $200 \leq D_2 \leq 800$, $5 \leq L_2 / D_2 \leq 15$, $20 \leq n_2 \leq 50$, $3 \leq D_2 / d_{21} \leq 10$, and $9 \leq D_2 / d_{22} \leq 20$.

**6.** The process according to any one of Claims 1 to 5, wherein each of said first continuous multi-stage distillation column and said second continuous multi-stage distillation column comprises a distillation column having a tray and / or a packing as said internal.

**7.** The process according to Claim 6, wherein said first continuous multi-stage distillation column comprises a plate type distillation column having a tray as said internal, and said second continuous multi-stage distillation column comprises a distillation column having both a packing and a tray as said internal.

**8.** The process according to Claim 6 or 7, wherein each of said trays in said first continuous multi-stage distillation column and said second continuous multi-stage distillation column is a sieve tray having a sieve portion and a downcomer portion.

**9.** The process according to Claim 8, wherein said sieve tray has 100 to 1000 holes / $m^2$ in said sieve portion.

**10.** The process according to Claim 8 or 9, wherein a cross-sectional area per one hole of said sieve tray is in a range of from 0.5 to 5 $cm^2$.

**11.** The process according to Claim 6 or 7, wherein said second continuous multi-stage distillation column comprises a distillation column having, as said internal, the packing in the upper portion of the column, and the tray in the lower

portion of the column.

**12.** The process according to any one of Claims 6 to 11, wherein the packing as said internal in said second continuous multi-stage distillation column is one or a plurality of sets of structured packings.

**13.** The process according to Claim 12, wherein said structured packing in said second continuous multi-stage distillation column is at least one type selected from the group consisting of Mellapak, Gempak, Techno-pack, Flexipac, a Sulzer packing, a Goodroll packing, and Glitschgrid.

**14.** The process according to any one of Claims 1 to 13, wherein the diaryl carbonate purification step (II) is carried out by distillation.

**15.** The process according to any one of Claims 1 to 14, wherein the side casing in the polymerization reaction zone comprises a cylindrical portion with an inside diameter D (cm) and a length L (cm), the tapered bottom casing, which is connected to a lower portion of the side casing, is conical, and the discharge port, which is in a lowermost portion of said conical casing, is cylindrical with an inside diameter d (cm), wherein D, L and d satisfy the following formulae (20), (21), (22) and (23),

$$100 \leq D \leq 1800 \qquad (20),$$

$$5 \leq D / d \leq 50 \qquad (21),$$

$$0.5 \leq L / D \leq 30 \qquad (22),$$

and

$$h - 20 \leq L \leq h + 300 \qquad (23).$$

**16.** The process according to any one of Claims 1 to 15, wherein said h satisfies the following formula (24),

$$400 < h \leq 2500 \qquad (24).$$

**17.** The process according to any one of Claims 1 to 16, wherein one of said guides is cylindrical, or pipe-shaped and made to be such that the molten prepolymer cannot enter therein, with an outside diameter r (cm), wherein r satisfies the following formula (25),

$$0.1 \leq r \leq 1 \qquad (25).$$

**18.** The process according to any one of Claims 1 to 17, wherein the polymerization is carried out using a plurality of said guide-contacting downflow type polymerization apparatuses linked together.

**19.** The process according any one of Claims 1 to 18, wherein the plurality of the guide-contacting downflow type polymerization apparatuses according to Claim 18 comprise two polymerization apparatuses being a first guide-contacting downflow type polymerization apparatus and a second guide-contacting downflow type polymerization apparatus, wherein in a process in which a polymerization degree is increased in this order, a total external surface area S1 ($m^2$) of the guides in said first guide-contacting downflow type polymerization apparatus and a total external

surface area S2 ($m^2$) of the guides in said second guide-contacting downflow type polymerization apparatus satisfy the following formula (26),

$$1 \leq S1 / S2 \leq 20 \qquad (26).$$

**20.** A high-quality aromatic polycarbonate produced in an amount of not less than 1 ton / hr by the process according to any one of Claims 1 to 19.

**21.** The high-quality aromatic polycarbonate according to claim 20, having a content of alkali metal and / or alkaline earth metal compounds in a range of from 0.1 to 0.01 ppm in terms of metallic elements therein, and a halogen content of not more than 1 ppb.

**22.** The high-quality aromatic polycarbonate according to claim 20 or 21, being an aromatic polycarbonate having a main chain thereof partially branched through foreign linkages such as an ester linkage or a ether linkage, and having a content of the foreign linkage in a range of from 0.05 to 0.5 mol% based on carbonate linkage.

# FIG.1

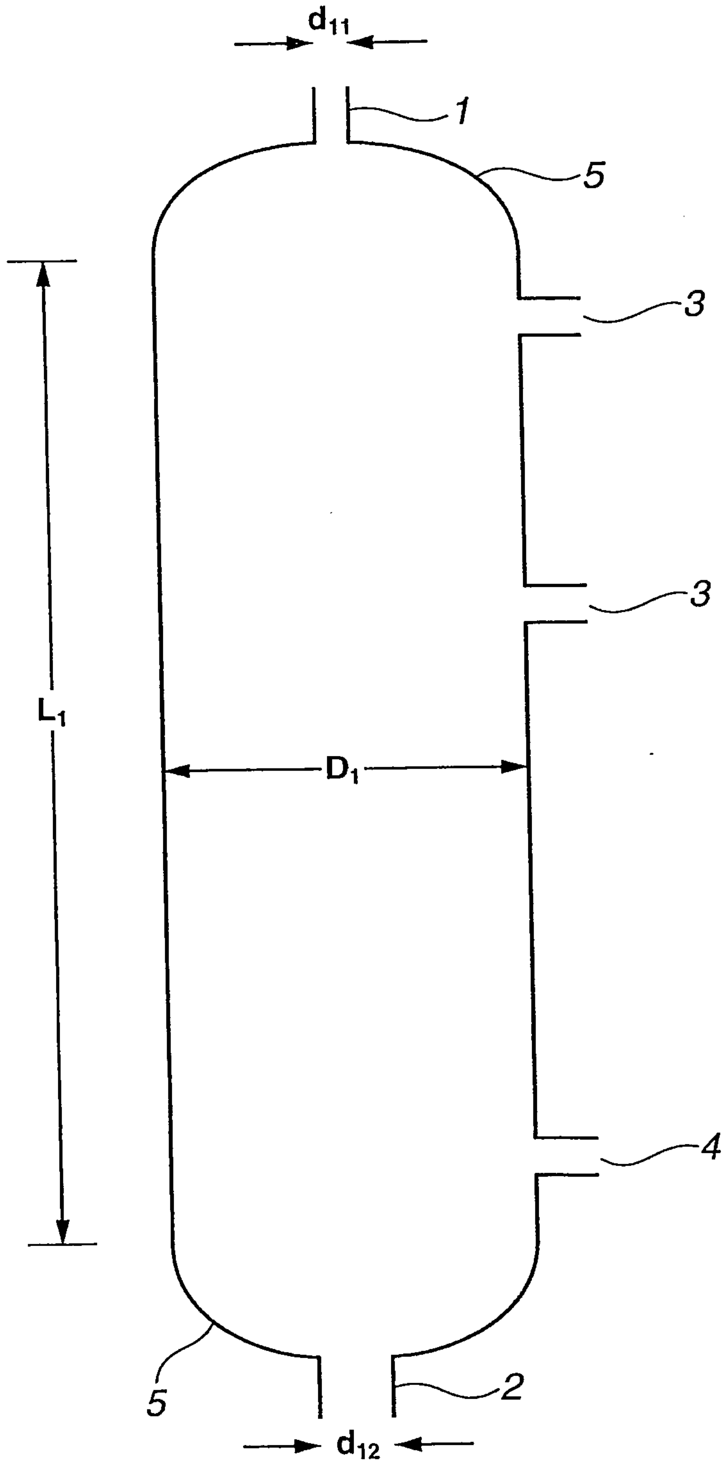

FIG.2

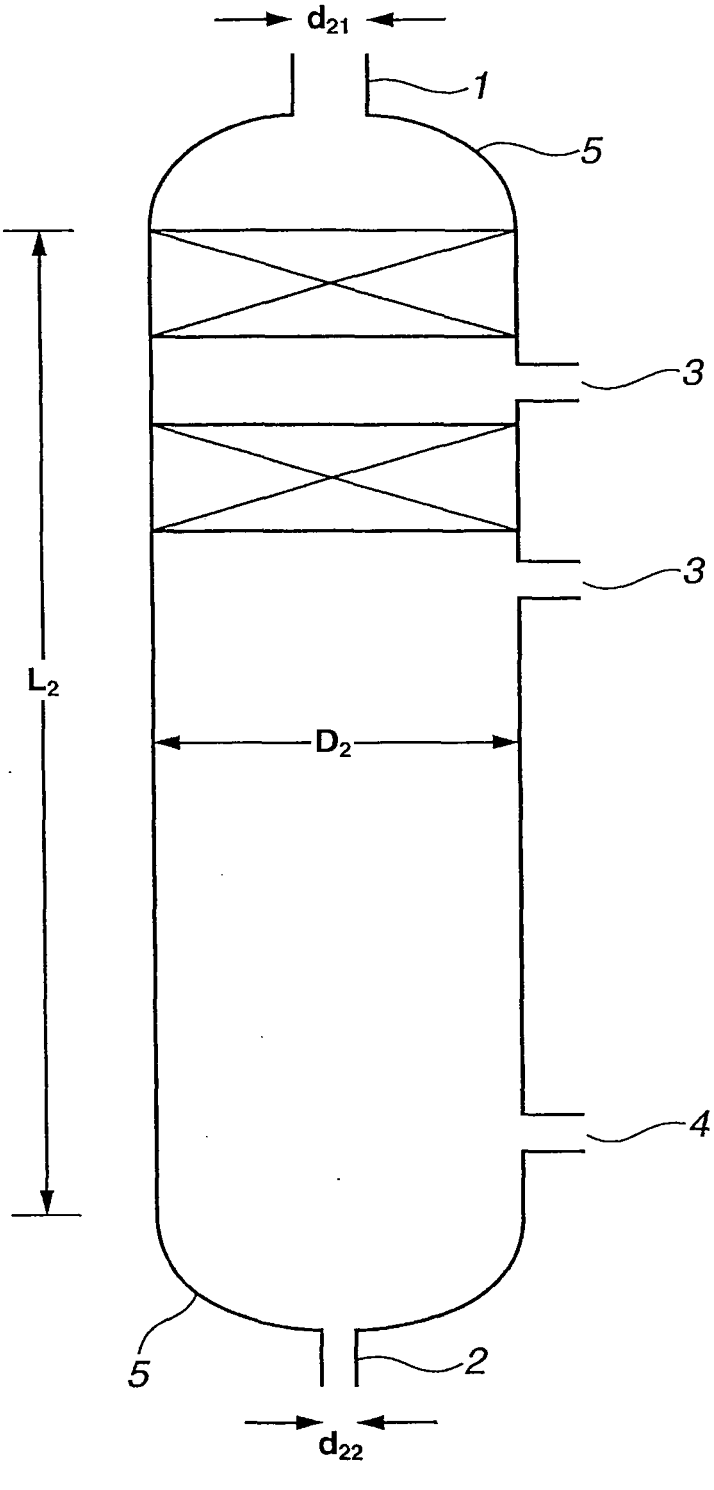

FIG.3

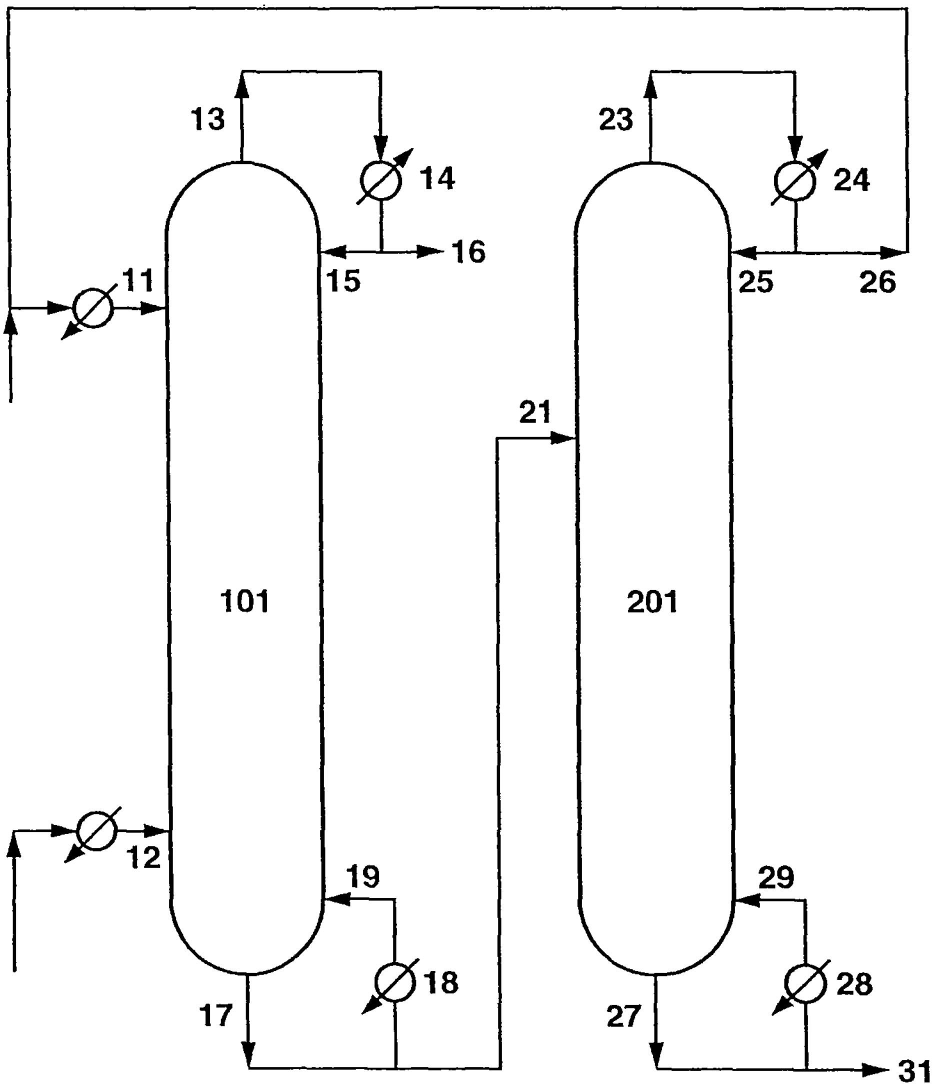

FIG.4

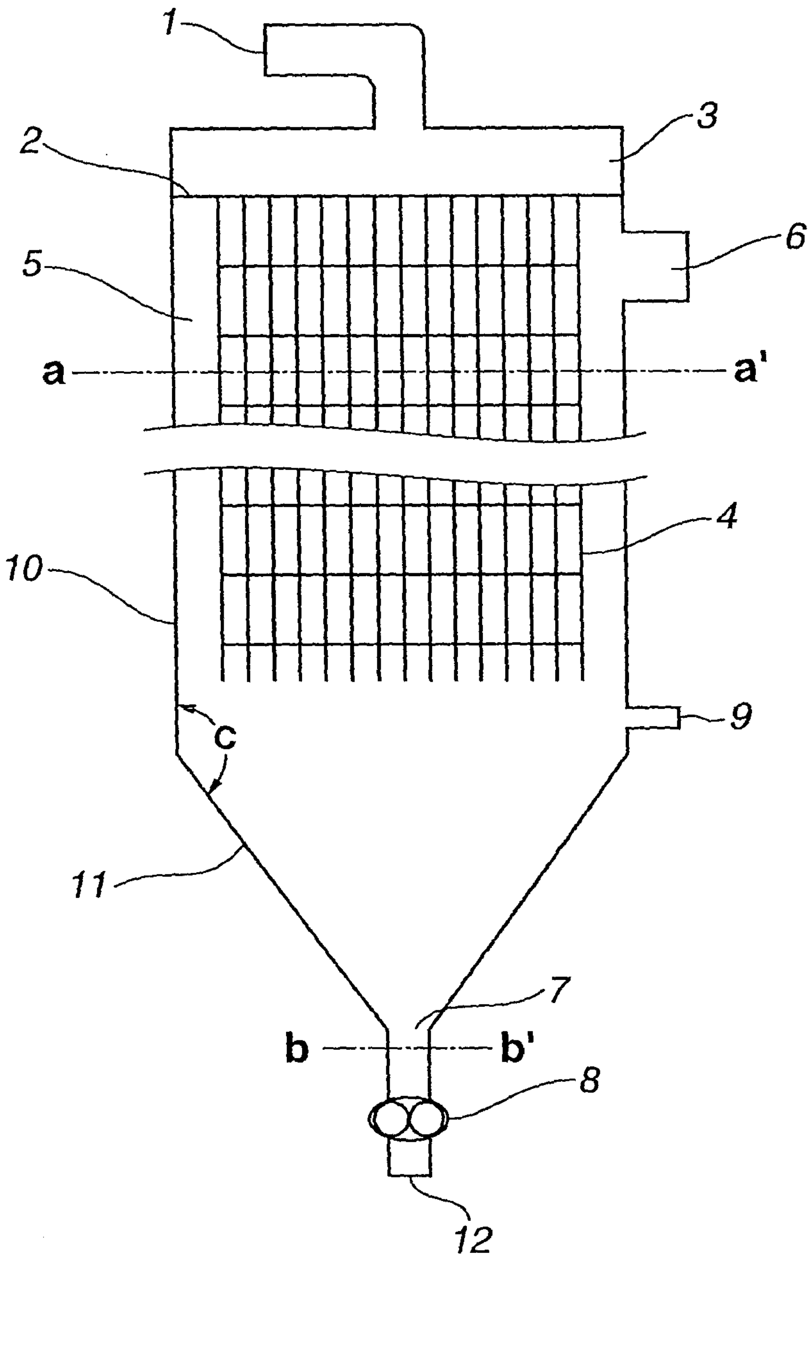

# FIG.5

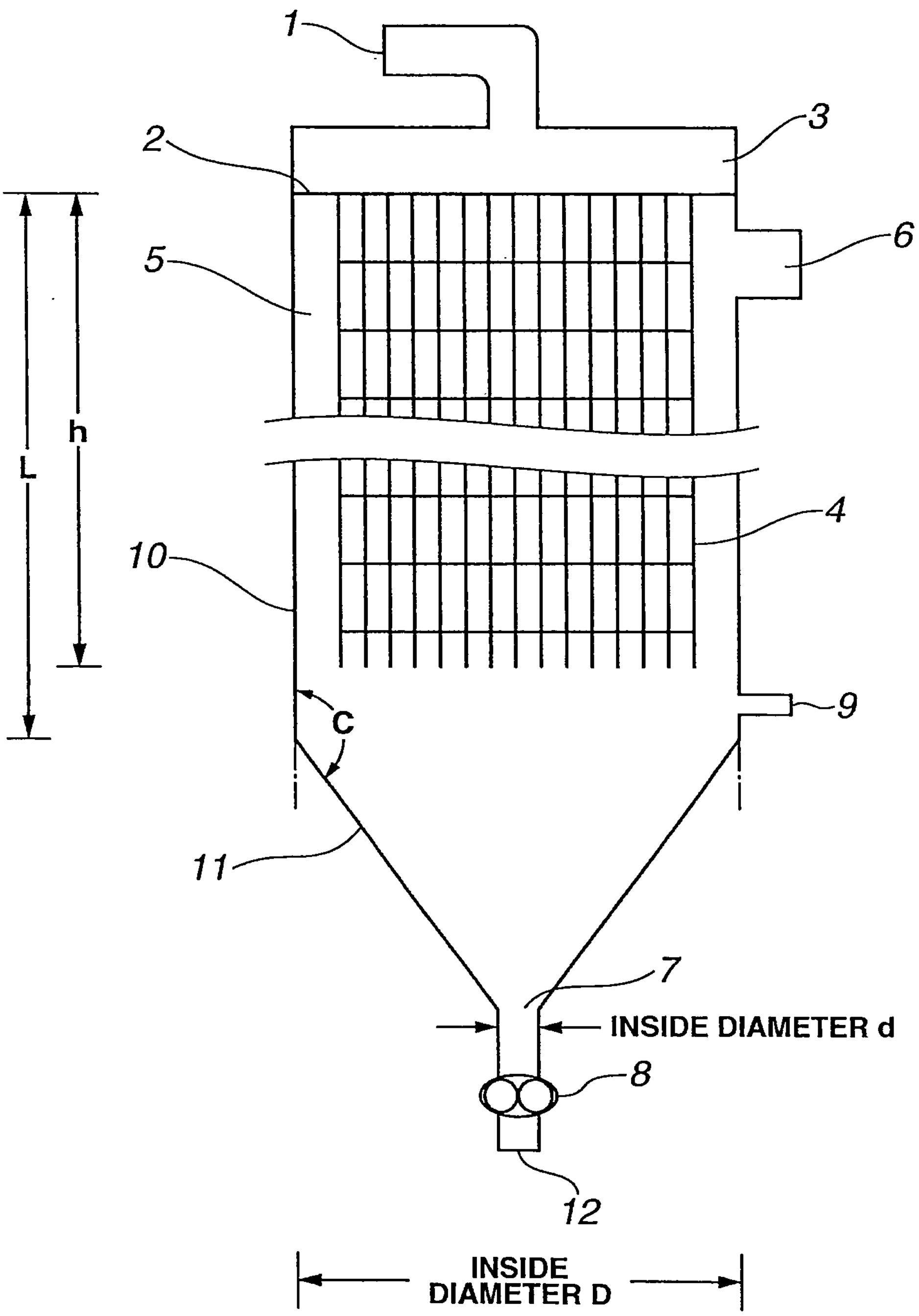

## INTERNATIONAL SEARCH REPORT

International application No. PCT/JP2006/323321

A. CLASSIFICATION OF SUBJECT MATTER
*C08G64/30*(2006.01)i, *B01D3/14*(2006.01)i, *B01D3/22*(2006.01)i, *C07C68/06*(2006.01)i, *C07C69/96*(2006.01)i

According to International Patent Classification (IPC) or to both national classification and IPC

B. FIELDS SEARCHED

Minimum documentation searched (classification system followed by classification symbols)
C08G64/30, B01D3/14, B01D3/22, C07C68/06, C07C69/96

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
WPIL(QWEB)

C. DOCUMENTS CONSIDERED TO BE RELEVANT

| Category* | Citation of document, with indication, where appropriate, of the relevant passages | Relevant to claim No. |
|---|---|---|
| X | JP 2005-146050 A (Mitsubishi Chemical Corp.), 09 June, 2005 (09.06.05), Claims; Par. Nos. [0062], [0066], [0072], [0078] & WO 2005/19302 A & EP 1657272 A | 20-22 |
| X | JP 9-255772 A (Asahi Chemical Industry Co., Ltd.), 30 September, 1997 (30.09.97), Claims; Par. Nos. [0049], [0050], [0058] & US 5747609 A & EP 892001 B & SG 52906 A & TW 442516 A & CN 1205344 A | 20-22 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 December, 2006 (21.12.06) | 09 January, 2007 (09.01.07) |
| Name and mailing address of the ISA/ Japanese Patent Office | Authorized officer |
| Facsimile No. | Telephone No. |

Form PCT/ISA/210 (second sheet) (April 2005)

**INTERNATIONAL SEARCH REPORT**

International application No.
PCT/JP2006/323321

C (Continuation). DOCUMENTS CONSIDERED TO BE RELEVANT

| Category* | Citation of document, with indication, where appropriate, of the relevant passages | Relevant to claim No. |
|---|---|---|
| X | JP 9-165443 A (Jemu Pi Shi Kabushiki Kaisha), 24 June, 1997 (24.06.97), Claims; example 1 & EP 779312 B & US 5760156 A & SG 44995 A & CN 1160062 A | 20-22 |
| A | JP 2004-211107 A (Asahi Kasei Chemicals Corp.), 29 July, 2004 (29.07.04), Claims & WO 99/64492 A & EP 1095957 B & US 6320015 B & CN 1294606 A & KR 2001052508 A | 1-22 |
| A | JP 2002-226573 A (Bayer AG.), 14 August, 2002 (14.08.02), Claims & EP 1221454 B & US 6703473 B & BR 200200008 A & CN 1367190 A & KR 2002057827 A & SG 102022 A & TW 574257 A | 1-22 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP S5019600 B
- GB 1007302 A
- JP S5236159 B
- JP S535718 B
- US 3888826 A
- JP H2153923 B
- JP H8225641 B
- JP H8225643 B
- JP H8325373 B
- WO 9722650 A
- JP H1081741 B
- JP H10298279 B
- WO 9936457 A
- WO 9964492 A
- JP S5448732 B
- DE 736063
- US 4252737 A
- JP S58185536 B
- US 410464 A
- JP S56123948 B
- US 4182726 A
- JP H3291257 B
- JP H49358 A
- JP H4211038 B
- WO 9109832 A
- EP 0461274 A
- US 5210268 A
- JP H4235951 B
- JP H6157424 B
- EP 0582931 A
- US 5334742 A
- JP H6184058 B
- EP 0582930 A
- US 5344954 A
- JP H940616 B
- JP H959225 B
- JP H9176094 B
- WO 0018720 A
- US 6093842 A
- JP 2001064235 A
- WO 9711049 A
- EP 0855384 A
- US 5872275 A
- JP H1192429 B
- EP 1016648 A
- US 6262210 B
- JP H9255772 B
- EP 0892001 A
- US 5747609 A
- WO 9732916 A